# EUROPÄISCHE PATENTANMELDUNG

(11) **EP 3 262 942 A1**
(43) Veröffentlichungstag der Anmeldung: **03.01.2018**
(21) Anmeldenummer: 16176413.9
(22) Anmeldetag: 27.06.2016
(51) Int. Cl.: A01N 59/02, A61K 31/095, C07C 391/02, A01P 3/00

(54) **SELENODIARYLE ZUR ANWENDUNG ALS MIKROBIZID SOWIE ALS ARZNEIMITTEL**

(71) Anmelder: Evonik Degussa GmbH, 45128 Essen (DE)
(72) Erfinder: DYBALLA, Katrin Marie,, 45657 Recklinhausen, (DE); FRANKE, Robert,, 45772 Marl, (DE); FRIDAG, Dirk,, 45721 Haltern am See, (DE); WALDVOGEL, Siegfrid R.,, 55435 Gau-Algesheim, (DE); THINES, Eckhard,, 67678 Mehlingen, (DE); QUELL, Thomas,, 45657 Recklinghausen (DE)

(57) **Zusammenfassung**

Selenodiaryle zur Anwendung als Mikrobizid oder als Arzneimittel sowie Zusammensetzungen enthaltend mindesten ein Selenodiaryl.

## Beschreibung

Selenodiaryle zur Anwendung als Mikrobizid sowie zur Anwendung als Arzneimittel und Zusammensetzungen enthaltend mindesten ein Selenodiaryl.

Pathogene wie Pilze sind in der Natur weit verbreitet. Durch einen Pilzbefall kommt es zur Schädigung von Nutzpflanzen. Dies kann zu massiven Ernteausfällen in der Landwirtschaft führen. Zudem kann ein Pilzbewuchs erhebliche Schäden an Bauwerken verursachen. Dabei können die Bausubstanzen, wie Hölzer, direkt befallen werden. Es kann aber auch zu indirekten Schäden kommen, indem Dicht- und Isolationsmaterialien geschwächt werden und so der Schutz vor äußeren Einflüssen nicht mehr gewährleistet werden kann. Durch Eindringen von Wasser kann so die Zersetzung der Bausubstanz durch weiteren Pilzbefall erhöht werden. Der zunehmende Einsatz von Außendämmungen in Deutschland führt dazu, dass in diesem Bereich mehr Schutz vor Pilzbefall benötigt wird. Solche Dämmungen führen zu einem schlechteren Feuchtigkeitsaustausch und können z.B. zu Schimmel in und am Haus führen. Pilzbefall in Innenräumen kann zu erheblichen gesundheitlichen Probleme bei Menschen führen, da diese zum Teil hochtoxische Mykotoxine freisetzten, oder ihre Sporen bis in die Lunge vordringen. Die Entwicklung neuartiger Fungizide zum Schutz von Pflanzen und zum Einsatz im Bauwesen stellt daher einen wichtigen Forschungsbereich zur Zukunftssicherung dar. Entsprechendes gilt für eine fungizide Ausrüstung von Textilien, die in allen Lebensbereichen sowie im gewerblichen Bereichen zum Einsatz gelangen.

Zur Pilzbekämpfung haben sich eine Reihe von Wirkstoffen aus der Gruppe der Benzimidazol-Carbamate, wie Carbendazim etabliert. Dieser Wirkstoff wird sowohl im Agrarals auch im Baustoff-Bereich eingesetzt. Ein weiteres Fungizid aus der Gruppe der Carbamate ist lodocarb, welches, neben seinem Einsatz in Holzschutzprodukten auch in Silikonen eingesetzt werden kann, um das Wachstum von Pilzen zu hemmen. Im Bereich der Dichtmassen, besonders im Nassraumbereich werden zugesetzte Fungizide ausgewaschen, wodurch ein Langzeit Schutz erschwert wird. Daher wird versucht die Fungizide mittels Verkapselungstechniken zu verarbeiten. Dabei werden vor allem Isothiazolinone wie Octylisothiazolinon, Dichloroctylisothiazolinon eingesetzt. Neben den vorgenannten organischen Verbindungen wird auch Zink-Pyrithion im Bauchemikalienbereich verwendet. Auf Grund seiner UV-Beständigkeit findet es dabei bevorzugt Anwendung in Außenanstrichen um Häuserwände vor Schimmelbefall zu schützen sowie als Additiv in Tapeten und Latex-Farben, um Schimmelbefall in Innenräumen vorzubeugen.

Die vorgenannten Wirkstoffe weisen zwar eine Aktivität gegen Pathogene aus dem Bereich der Pilze auf, jedoch zeigen sie eine signifikante Wirkung gegen andere nützliche Organismen. So weist Carbendazim zwar keine akute Toxizität auf, doch Untersuchungen an Tieren zeigten, dass eine dauerhafte Aufnahme zu Schädigung von Leber und Nieren führen kann. Zudem deuten Studien darauf hin, dass das Risiko einer reproduktionstoxischen Wirkung besteht. Gerade in Bereichen, in denen Menschen dauerhaft mit Fungiziden in Kontakt kommen können besteht daher die Gefahr von Langzeitschäden. Die Verwendung von lodocarb erscheint hier sogar noch kritischer, da von diesem Stoff akute und chronische Gesundheitsgefahren ausgehen. Iodocarb ist zudem als gewässergefährdend eingestuft, wodurch es zur Gefährdung der Umwelt durch Auswaschungen kommen kann. Diese Einschränkungen gelten auch für Octylisothiazolinon, von dem ebenfalls akute und chronische Gesundheitsgefahren ausgehen sowie eine gewässergefährdende Wirkung. Zwar ist Zink-Pyrithion praktisch unlöslich in Wasser, dennoch kann auch dieser Stoff ausgewaschen werden, sodass die wassergefährdende Wirkung eine große Rolle spielt. Die akute Toxizität, sowie die chronische Gesundheitsgefährdung stellen einen großen Nachteil dieses Stoffes da.

Neben den derzeit verwendeten Fungiziden gibt es Untersuchungen zu selenhaltigen Verbindungen mit fungizider Wirkung. Die fungizide Wirkung ist für diese Verbindungen jedoch nur gering oder wird erst bei hohen Dosen erzielt. So wird für Selen enthaltende Aminosäureverbindungen (Sh. H. Abdel-Hafez et. al, Russion Journal of bioorganic Chemistry, 2011, Vol. 37, No. 3, pp. 261-269) keine fungizide Aktivität nachgewiesen oder alternativ nur für zwei Verbindungen eine fungizide Aktivität bei hohen Konzentrationen. Selenadiazole mit einer gewissen fungiziden Wirkung werden von A. R. Jalilian et al., in II Farmaco 58 (2003), 63-68 berichtet. Allerdings lassen sich aufgrund der Anwesenheit des Diazolstrukturelements keine Rückschlüsse auf dessen Aktivität oder auf die Notwendigkeit des Selens ziehen. Des Weiteren sind Selen enthaltende Tetrazole aus Francieli M. Libero et al., Tetrahedron Letters 53, 2012, 3091-3094 mit sehr geringer Aktivität bei hohen MIC (minimum inhibitory concentration [µM]) bekannt.

Aufgabe der Erfindung war es neue mikrobizide Wirkstoffe bereitzustellen. Insbesondere bestand die Aufgabe neue fungizide Wirkstoffe bereitzustellen. Diese Wirkstoffe sollen einerseits eine gute fungizide Wirkung aufweisen und vorteilhaft leicht mit einer Matrix, wie u.a. mineralische Substrate, funktionalisierbare Dichtungsmassen, Baustoffe oder Textilien, koppelbar sein, so dass ein Auswaschen und damit einer Abnahme der mikrobiziden Wirkung, insbesondere der fungiziden Wirkung, vorgebeugt und vorzugsweise unterbunden werden kann.

Des Weiteren sollen diese Wirkstoffe sehr wirtschaftlich herstellbar sein. Vorzugsweise sollen die Wirkstoffe mittels einer geringen Anzahl an Synthesestufen mit vorzugsweise kostengünstigen Edukten und insbesondere geringem Lösemitteleinsatz und hohen Ausbeuten herstellbar sein.

Gelöst werden die Aufgaben der Erfindung mit den Selenodiarylen der allgemeinen Formel I, die insbesondere zur Anwendung als Arzneimittel, vorzugsweise als Mikrobizid wirkendes Arzneimittel, oder zur Anwendung als Mikrobizid, vorzugsweise als Fungizid nach Anspruch 1 und mit Zusammensetzungen nach Anspruch 12 sowie deren Verwendung nach Anspruch 15 zur Herstellung einer mikrobizid wirkenden nicht medizinischen Ausrüstung oder zur Herstellung einer mikrobizid wirkenden nicht medizinischen Zusammensetzung und/oder zur mikrobizid wirkenden Funktionalisierung oder Ausrüstung von funktionalisierbaren Substraten. Bevorzugte Ausführungsformen werden in den Unteransprüchen sowie detailliert in der Beschreibung offenbart.

Gelöst werden die Aufgaben, indem ausgehend von Selendioxid eine Umsetzung mit Phenolderivaten in Pyridin selektiv zu Bis(hydroxyphenyl)selen-Verbindungen erfolgen kann. Mit dieser allgemeinen Synthese kann eine breite Klasse an Bis(hydroxyphenyl)selen-Verbindungen, wie Alkyl-substituierte oder auch halogenierte Phenole, umgesetzt werden. Bevorzugt sind bromierte, chlorierte und fluorierte Phenole. Die Verwendung von Essigsäure als Lösungsmittel ermöglicht zusätzlich die Herstellung von Diarylseleniden. Die ersten Untersuchungen auf eine mikrobizide Wirksamkeit zeigten, dass diese Selen-Verbindungen eine hohe Aktivität gegen Pilzerreger aufweisen. Die erfindungsgemäßen Selen-verbrückten Aromaten bilden somit eine neue Klasse an Wirkstoffen mit fungizider Aktivität. Die fungizide Wirkung konnte bereits gegenüber einer Vielzahl an Pilzen nachgewiesen werden. Erhältlich sind diese Wirkstoffe in der Regel über eine sehr wirtschaftliche einstufige Synthese. Daher können die erfindungsgemäßen Selen-Verbindungen äußerst kosteneffizient und einfach realisiert werden. Der Bedarf an Fungiziden zur Bekämpfung schädlicher Pilze ist sehr hoch, denn in vielen Produkten des täglichen Lebens sind Fungizide enthalten, um die Haltbarkeit bzw. die Beständigkeit zu erhöhen. Die Entwicklung und der Einsatz neuer Fungizide hat daher eine große wirtschaftliche Bedeutung. Die hohe Aktivität gegen verschiedene Pilzstämme erlaubt einen Einsatz als Breitbandfungizid in unterschiedlichen Produktbereichen. Die erfindungsgemäßen Selen-Verbindungen haben das Potential herkömmliche, zum Teil gefährliche Fungizide abzulösen. Alternativ können die Verbindungen auch in Wirkstoffkombinationen eingesetzt werden.

Gegenstand der Erfindung ist mindestens ein Selenodiaryl der allgemeinen Formel I zur Anwendung als Mikrobizid oder Arzneimittel, insbesondere als mikrobizid wirkendes Arzneimittel, insbesondere als nicht medizinisches bzw. nicht pharmazeutisches Mikrobizid, wobei das eine Selenodiaryl der allgemeinen Formel I umfasst, 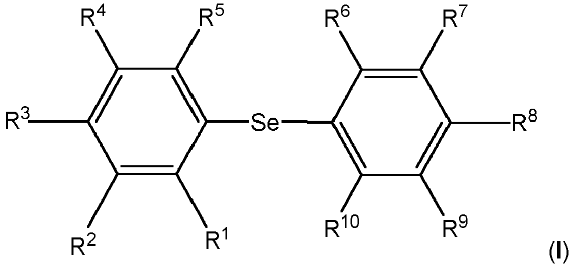 worin R¹, R², R³, R⁴, R⁵, R⁶, R¹, R⁸, R⁹ und R¹⁰ jeweils unabhängig voneinander ausgewählt sind aus: -H, -OH, -(C₁-C₁₂)-Alkyl, -O-(C₁-C₁₂)-Alkyl, -(C₆-C₂₀)-Aryl, -O-(C₆-C₂₀)-Aryl, -Halogen, -S-Alkyl, -S-Aryl, -CO-(C₁-C₁₂)-Alkyl, -CO-(C₆-C₂₀)-Aryl, -(C₁-C₁₂)-Alkyl-O-(C₁-C₁₂)-Alkyl, -(C₁-C₁₂)-Alkyl-(C₆-C₂₀)-Aryl, -(C₁-C₁₂)-Alkyl-O-(C₆-C₂₀)-Aryl, -(C₆-C₂₀)-Aryl-O-(C₆-C₂₀)-Aryl, -(C₆-C₂₀)-Aryl-(C₁-C₁₂)-Alkyl, -(C₁-C₁₂)-Alkyl-O-(C₁-C₁₂)-Alkyl-(C₆-C₂₀)-Aryl, -(C₁-C₁₂)-Alkyl-(O-(C₁-C₁₂)-Alkyl)ₓ-O-(C₁-C₁₂)-Alkyl, mit x = 1 bis 3, -COOH, -SO₃H, -CN, wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, -Halogen, -Cyano, -Formyl, -Acyl oder -Alkoxycarbonyl.

Besonders bevorzugt kann das Selenodiaryl der allgemeinen Formel **I** zur Anwendung als Arzneimittel oder als Mikrobizid, zur Anwendung als Biozid, insbesondere als Antimykotikum verwendet werden. Vorzugsweise kann das Selenodiaryl der Formel **I** zur prophylaktischen mikrobiziden Verwendung und/oder zur Verwendung bei der Behandlung von Mikrobiziden eingesetzt werden. Besonders bevorzugt ist das Selenodiaryl der Formel **I** geeignet zur prophylaktischen antimykotischen oder fungiziden Verwendung und/oder zur Verwendung bei der Behandlung von Pilzbefall, Mykosen oder Pilzerkrankungen. Das Selenodiaryl der Formel I weist erfindungsgemäß eine antimykotische Wirkung auf. Des Weiteren ist es bevorzugt, wenn das Selenodiaryl der Formel **I** die Sporenbildung, das Hyphen-, Myzel- und/oder Mykorrizawachstum mindestens eines Pilzes, insbesondere umfassend resistente Pilze, zumindest hemmt, reduziert oder verhindert und/oder den mindestens einen Pilz abtötet.

Bevorzugt ist das Selenodiaryl der allgemeinen Formel **I** zur Anwendung als fungizides Arzneimittel oder zur Anwendung als Fungizid, vorzugsweise zur Anwendung als Breitband-Antimykotikum geeignet, wobei der mindestens eine Pilz ausgewählt ist aus der Ordnung *[Mucorales, Peronosporales, Eurotiales, Helotiales, Magnaporthales und*/*oder Saccharomycetales*]. Nach einer weiteren Ausführungsform weist das Selenodiaryl der allgemeinen Formel **I** eine fungistatische und/oder fungizide Wirkung auf.

Bevorzugt ist das Selenodiaryl der allgemeinen Formel I zur Anwendung als fungizides Arzneimittel oder zur Anwendung als Fungizid, insbesondere zur Anwendung als Antimykotikum gegenüber mindestens einem Pilz der nachfolgenden Gattung, bevorzugt zur Anwendung als Breitband-Antimykotikum, wobei der mindestens eine Pilz ausgewählt ist aus der Gattung *Mucor, Phytophthera, Paecilomyces, Penicillium, Botrytis, Magnaporthe* und/oder *Candida.* Nach einer weiteren Ausführungsform weist das Selenodiaryl der allgemeinen Formel **I** eine fungistatische und/oder fungizide Wirkung auf.

Mikrobizid ist die Eigenschaft einer Substanz, d.h. eines Mikrobizids, Mikroorganismen zu schädigen bzw. abzutöten. Richtet sich die Wirkung nur gegen eine spezielle Art von Mikroorganismen, unterscheidet man zwischen algizid (Algen), bakterizid/bakteriostatisch (Bakterien), fungizid /fungistatisch (Pilze) oder viruzid (Viren). Mikrobizide umfassen Biozide. In vielen Dispersionen wie Farben, Lacken, Reinigungsmitteln, Shampoos und Kosmetika sind Mikrobizide notwendige Inhaltsstoffe (sog. Topfkonservierer), um eine lange Haltbarkeit und eine gute Produktqualität zu gewährleisten.

Die erfindungsgemäßen Mikrobizide, insbesondere Fungizide können vorzugsweise in der Landwirtschaft als Pflanzenschutzmittel angewendet werden. Daneben können sie zur Bekämpfung von Schadpilzen (z. B. Schimmelpilzen) auf Holz, Farbe, Textilien, an Wänden (Hausschwamm) und bei Lebensmitteln dienen. Für Fungizide, die in der Medizin (z. B. gegen Hautpilze) eingesetzt werden, ist der Begriff Antimykotika gebräuchlicher. Im Gegensatz zur Landwirtschaft werden auch fungizid wirkende Biozide als Desinfektionsmittel eingesetzt

Gleichfalls Gegenstand der Erfindung ist ein Selenodiaryl zur Anwendung als Arzneimittel, insbesondere als mikrobizid wirkendes Arzneimittel, oder als Mikrobizid, wobei das Selenodiaryl der allgemeinen Formel **I** in einer Zusammensetzung mit einem Gehalt größer gleich 0,0001 Gew.-% bis 100 Gew.-% vorliegt, insbesondere von 0,0001 Gew.-% bis 60 Gew.-%, bevorzugt von 0,0001 Gew.-% bis 40 Gew.-%, besonders bevorzugt von 0,0001 Gew.-% bis 20 Gew.-%, vorzugsweise von 0,0001 Gew.-% bis 10 Gew.-%, alternativ von 0,0001 Gew.-% bis 5 Gew.-% oder von 0,0001 Gew.-% bis 1 Gew.-%, wobei die Gesamtzusammensetzung 100 Gew.-% beträgt.

Vorzugsweise liegt die MIC (minimum inhibitory concentration [µM]) der Selenodiaryle der allgemeinen Formel I bei größer gleich 0,001 bis 50 µg/mL, insbesondere bei größer gleich 0,001 bis 30 µg/mL, bevorzugt bei größer gleich 0,01 bis 25 µg/mL, besonders bevorzugt bei größer gleich 0,01 bis 15 µg/mL, weiter bevorzugt bei größer gleich 0,01 bis 10 µg/mL. Besonders bevorzugt liegt die MIC bei größer gleich 0,01 bis 5 µg/mL, insbesondere jeweils unabhängig mit +/- 0,5 µg/mL, vorzugsweise mit +/- 0,25 µg/m.

Vorzugsweise weist mindesten ein Selenodiaryl der allgemeinen Formel I eine fungistatische und/oder fungizide Wirkung auf bei einem Gehalt von größer gleich 0,1 µg/ml, vorzugsweise größer gleich 0,5 µg/ml, größer gleich 1 µg/ml, besonders bevorzugt größer gleich 5 µg/ml, insbesondere von 1 µg/ml bis kleiner gleich 1000 µg/ml.

Entsprechend einer besonders bevorzugten Ausführungsform kann das Selenodiaryl der allgemeinen Formel I ausgewählt sein aus der allgemeinen Formel **II,** vorzugsweise zur Anwendung als Breitband-Antimykotikum, 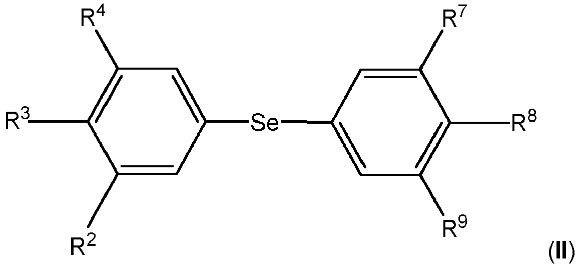 worin R², R⁴, R⁷ und R⁹ jeweils unabhängig voneinander ausgewählt sind aus: -H, -OH, -(C₁-C₁₂)-Alkyl, -O-(C₁-C₁₂)-Alkyl, -(C₆-C₂₀)-Aryl, -O-(C₆-C₂₀)-Aryl, -Halogen, -(C₁-C₁₂)-Alkyl-O-(C₁-C₁₂)-Alkyl, -(C₁-C₁₂)-Alkyl-(C₆-C₂₀)-Aryl, -(C₁-C₁₂)-Alkyl-O-(C₆-C₂₀)-Aryl, -(C₆-C₂₀)-Aryl-O-(C₆-C₂₀)-Aryl, -(C₆-C₂₀)-Aryl-(C₁-C₁₂)-Alkyl, -(C₁-C₁₂)-Alkyl-O-(C₁-C₁₂)-Alkyl-(C₆-C₂₀)-Aryl, -(C₁-C₁₂)-Alkyl-(O-(C₁-C₁₂)-Alkyl)ₓ-O-(C₁-C₁₂)-Alkyl, mit x = 1 bis 3, wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Halogen, und,
worin R³ und R⁸ jeweils unabhängig voneinander ausgewählt sind aus: -OH, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkyl- und Arylgruppen jeweils unsubstituiert sind.

Im Selenodiaryl der Formel **II** sind R³ und R⁸ vorzugsweise jeweils unabhängig ausgewählt aus -OH, und/oder -Halogen, und R², R⁴, R⁷ und R⁹ sind jeweils unabhängig wie vorstehend definiert. Vorzugsweise mit R², R⁴, R⁷ und R⁹ jeweils unabhängig voneinander ausgewählt aus: -H, -OH, -(C₁-C₁₂)-Alkyl, insbesondere -(C₁-C₄)-Alkyl, vorzugsweise -(C₁-C₃)-Alkyl, -O-(C₁-C₁₂)-Alkyl, insbesondere -(C₁-C₄)-Alkyl, vorzugsweise -(C₁-C₃)-Alkyl, -Halogen, wobei die Alkylgruppen jeweils unabhängig unsubstituiert sind.

Des Weiteren sind im Selenodiaryl der Formel **II** R³ und R⁸ vorzugsweise jeweils unabhängig ausgewählt aus -OH und R², R⁴, R⁷ und R⁹ sind jeweils unabhängig voneinander ausgewählt aus: -H, -OH, -(C₁-C₄)-Alkyl, vorzugsweise -(C₁-C₃)-Alkyl, -Halogen, wobei die Alkylgruppen jeweils unabhängig unsubstituiert sind. Ferner sind im Selenodiaryl der Formel II R³ und R⁸ vorzugsweise jeweils unabhängig ausgewählt aus -OH und R², R⁴, R⁷ und R⁹ sind jeweils unabhängig voneinander ausgewählt aus: -OH, -(C₁-C₄)-Alkyl, vorzugsweise -(C₁-C₃)-Alkyl, wobei die Alkylgruppen jeweils unabhängig unsubstituiert sind. Besonders bevorzugt ist Bis(3,5-dimethyl-4-hydroxyphenyl)selen (19), wobei diese Verbindung insbesondere für eine Anwendung als Biozid, Desinfektionsmittel, Werkstoffschutzmittel, Medizinprodukt geeignet ist.

Gleichfalls kann das Selenodiaryl der Formel I zur Anwendung als Arzneimittel, insbesondere als mikrobizid wirkendes Arzneimittel, oder als Mikrobizid ausgewählt sein aus der allgemeinen Formel **Ia** 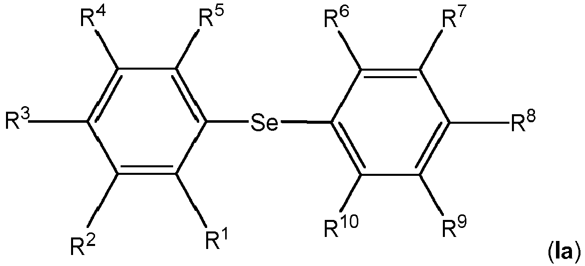 worin R¹, R³, R⁵, R⁶, R⁸ und R¹⁰ jeweils unabhängig voneinander ausgewählt sind aus: -H, -OH, -(C₁-C₁₂)-Alkyl, -O-(C₁-C₁₂)-Alkyl, -Halogen,
-(C₁-C₁₂)-Alkyl-O-(C₁-C₁₂)-Alkyl, -(C₁-C₁₂)-Alkyl-O-(C₁-C₁₂)-Alkyl-(C₆-C₂₀)-Aryl,
-(C₁-C₁₂)-Alkyl-(O-(C₁-C₁₂)-Alkyl)ₓ-O-(C₁-C₁₂)-Alkyl, mit x = 1 bis 3, wobei die Alkylgruppen jeweils unabhängig unsubstituiert oder substituiert sind, wobei substituierte -(C₁-C₁₂)-Alkylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, -Halogen, und, worin R², R⁴, R⁷ und R⁹ jeweils unabhängig voneinander ausgewählt sind aus: -H, -OH, -(C₁-C₄)-Alkyl, -O-(C₁-C₄)-Alkyl, -Halogen, wobei die Alkylgruppen jeweils unsubstituiert oder substituiert sind, wobei substituierte -(C₁-C₄)-Alkylgruppen mindestens einen Substituenten aufweisen und der mindestens eine Substituent jeweils unabhängig ausgewählt ist aus -Halogen.

Besonders bevorzugte Selenodiaryle der Formel **Ia** umfassen Strukturen mit R¹, R³, R⁵, R⁶, R⁸ und R¹⁰, die jeweils unabhängig voneinander ausgewählt sind aus: -H, -OH, -(C₁-C₁₂)-Alkyl, insbesondere -(C₁-C₄)-Alkyl, bevorzugt -(C₁-C₃)-Alkyl; -O-(C₁-C₁₂)-Alkyl, wobei die Alkylgruppen jeweils unsubstituiert sind und, worin R², R⁴, R⁷ und R⁹ jeweils unabhängig voneinander ausgewählt sind aus: -OH, -(C₁-C₄)-Alkyl; bevorzugt -(C₁-C₃)-Alkyl, besonders bevorzugt -(C₁-C₂)-Alkyl; -O-(C₁-C₄)-Alkyl, bevorzugt -O-(C₁-C₃)-Alkyl, -Halogen, wobei die Alkylgruppen jeweils unsubstituiert sind.

Besonders bevorzugte Selenodiaryl der Formel **Ia** umfassen R¹, R³, R⁵, R⁶, R⁸ und R¹⁰, die jeweils unabhängig voneinander ausgewählt sind aus: -H, -OH, -(C₁-C₄)-Alkyl, insbesondere -(C₁-C₃)-Alkyl, bevorzugt -(C₁-C₂)-Alkyl, -Halogen, wobei die Alkylgruppen jeweils unsubstituiert sind und, worin R², R⁴, R⁷ und R⁹ jeweils unabhängig voneinander ausgewählt sind aus: -OH, -(C₁-C₄)-Alkyl, bevorzugt -(C₁-C₃)-Alkyl, -O-(C₁-C₄)-Alkyl, bevorzugt -O-(C₁-C₃)-Alkyl, -Halogen, wobei die Alkylgruppen jeweils unsubstituiert sind.

Besonders bevorzugte Selenodiaryle der Formel **Ia** umfassen R¹, R³, R⁵, R⁶, R⁸ und R¹⁰, die jeweils unabhängig voneinander ausgewählt sind aus: -H, -OH, -(C₁-C₃)-Alkyl, wobei die Alkylgruppen jeweils unsubstituiert sind und, worin R², R⁴, R⁷ und R⁹ jeweils unabhängig voneinander ausgewählt sind aus: -H, -OH, -(C₁-C₃)-Alkyl, bevorzugt -(C₁-C₂)-Alkyl, -O-(C₁-C₃)-Alkyl, besonders bevorzugt -O-(C₁-C₂)-Alkyl, -Halogen, wobei die Alkylgruppen jeweils unsubstituiert sind. Des Weitern ist es bevorzugt, wenn mindestens ein R² oder R⁴ und R⁷ oder R⁹ jeweils unabhängig voneinander ausgewählt ist aus: -(C₁-C₃)-Alkyl oder -O-Methyl und das verbleibende R² oder R⁴ und R⁷ oder R⁹ jeweils ausgewählt ist aus -H, -OH, -(C₁-C₂)-Alkyl, -OMethyl oder -Halogen.

Besonders bevorzugte Selenodiaryle der Formel **Ia** umfassen R¹, R², R⁴, R⁵, R⁶, R¹, R⁹ und R¹⁰ die jeweils unabhängig voneinander ausgewählt sind aus: -H, -(C₁-C₁₀)-Alkyl, insbesondere -(C₁-C₅)-Alkyl, besonders bevorzugt -(C₁-C₄)-Alkyl, -O-(C₁-C₃)-Alkyl und/oder -Halogen, und R³ und R⁸ jeweils unabhängig ausgewählt sind aus -H, -OH, und/oder -O-(C₁-C₃)-Alkyl. Besonders bevorzugte Selenodiaryle der Formel **I umfassen** R¹, R², R⁴, R⁵, R⁶, R⁷, R⁹ und R¹⁰ die jeweils unabhängig voneinander ausgewählt sind aus: -H -(C₁-C₄)-Alkyl, insbesondere -(C₁-C₂)-Alkyl, -O-(C₁-C₄)-Alkyl, insbesondere -OMethyl, und/oder -Halogen, insbesondere -Cl, -F, -Br, und R³ und R⁸ jeweils -H sind, wie in der nachfolgenden allgemeinen Formel **III.**

Besonders bevorzugte Selenodiaryle der Formel **Ia** umfassen R², R³, R⁴, R⁵, R⁶, R¹, R⁸ und R⁹ die jeweils unabhängig voneinander ausgewählt sind aus: -H, -(C₁-C₃)-Alkyl und/oder -Halogen, und R¹ und **R¹⁰** jeweils -OH sind. Besonders bevorzugte Selenodiaryle der Formel I umfassen R¹, R², R⁴, R⁵, R⁶, R⁷, R⁹ und **R¹⁰** die jeweils unabhängig voneinander ausgewählt sind aus: -H -(C₁-C₄)-Alkyl, insbesondere -(C₁-C₂)-Alkyl und/oder -Halogen, und R³ und R⁸ jeweils -OH sind.

Besonders bevorzugte Selenodiaryle der Formel **Ia** umfassen R², R³, R⁴, R⁵, R⁶, R⁷, R⁸ und R⁹ die jeweils unabhängig voneinander ausgewählt sind aus: -H, -(C₁-C₄)-Alkyl, insbesondere -Methyl, - Ethyl, -iso-Propyl, n-Propyl, und/oder -Halogen, und R¹ und R¹⁰ jeweils -OH sind.

Besonders bevorzugte Selenodiaryle der nachfolgenden Formel **III** umfassen R², R⁴, R⁷ und R⁹ die jeweils unabhängig voneinander ausgewählt sind aus: -(C₁-C₄)-Alkyl, insbesondere - Methyl, und/oder -Halogen.

Besonders bevorzugte Selenodiaryle der nachfolgenden Formel **III** umfassen R², R⁵, R⁶ und R⁹ die jeweils unabhängig voneinander ausgewählt sind aus: -(C₁-C₄)-Alkyl, insbesondere -Methyl, und R⁴ und R⁷ die jeweils unabhängig voneinander ausgewählt sind aus: -Halogen.

Besonders bevorzugte Selenodiaryle sind Selenodiaryle der Formeln **III** und **IV** gemäß den Tabellen 1 und 2.

**Tabelle 1: Selenodiaryle der Formel III mit R¹ und R¹⁰ gleich -OH**

| **Produkt** | **Reste** | |
|---|---|---|
| 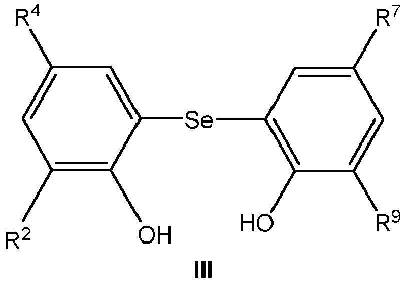 | R², R⁹ | R⁴, R⁷ |
| 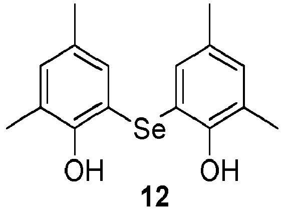 | -Me | -Me |
| 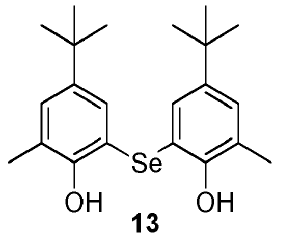 | -Me | -tBu |
| 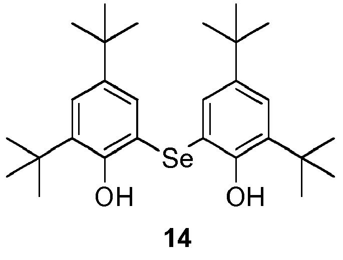 | -tBu | -tBu |
| 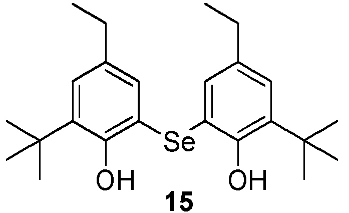 | -tBu | -Et |
| 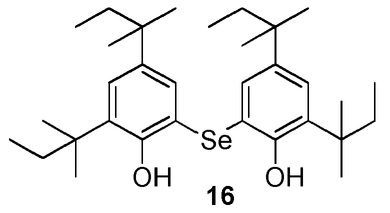 | -tPe | -tPe |
| 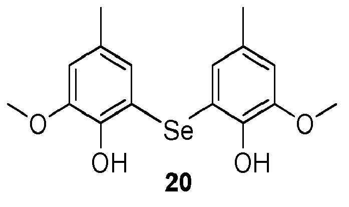 | -O-Me | -Me |
| 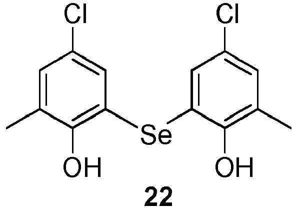 | -Me | -Cl |
| 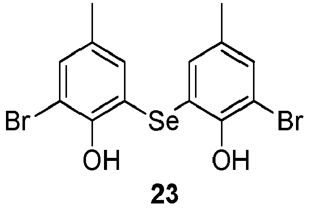 | -Br | -Me |
| 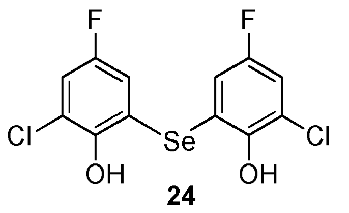 | -Cl | -F |
| 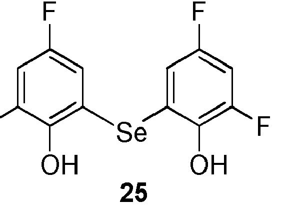 | -F | -F |

| | | |
|---|---|---|
| Me = Methyl, Et = Ethyl, iPr =*iso*-Propyl, tBu = *tert*.-Butyl, tPe = *tert*.-Pentyl | | |

**Tabelle 2: Selenodiaryle der Formel IV mit R¹ und R¹⁰ gleich -OH**

| **Produkt** | **Reste** | | |
|---|---|---|---|
| 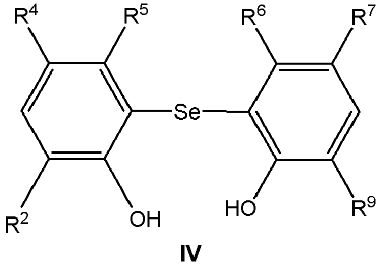 | R², R⁹ | R⁴, R⁷ | R⁵ R⁶ |
| 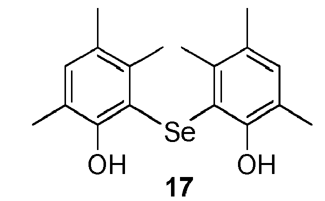 | -Me | -Me | -Me |
| 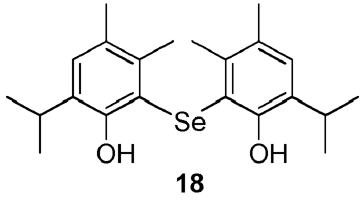 | -iPr | -Me | -Me |
| 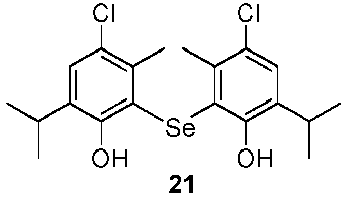 | -iPr | -Cl | -Me |

| | | | |
|---|---|---|---|
| Me = Methyl, iPr = iso-Propyl | | | |

Besonders bevorzugt sind Bis(3,5-dimethyl-2-hydroxyphenyl)selen (12), Bis(5-*tert*-butyl-2-hydroxy-3-methylphenyl)selen (13), Bis(2-hydroxy-3-methoxy-5-methylphenyl)selen (20), Bis(3-chlor-6-hydroxy-5-isopropyl-2-methylphenyl)selen (21), Bis(3-chlor-6-hydroxy-5-methylphenyl)selen (22), Bis(3-brom-2-hydroxy-5-methylphenyl)selen (23), da diese Verbindungen keine Schädigung von Nutzpflanzen verursachen. Daher sind Selenodiaryle der Formeln **III** und **IV,** insbesondere mit entsprechend vorgenanntem Substitutionsmuster besonders bevorzugt.

Ebenfalls besonders gut geeignet sind Bis(2-hydroxy-3,5,6-trimethylphenyl)selen (17), Bis(3-chlor-5-fluor-2-hydroxyphenyl)selen (24), Bis(3,5-difluoro-2-hydroxyphenyl)selen (25), wobei diese Verbindungen für eine Anwendung als Biozid, Desinfektionsmittel, Werkstoffschutzmittel, Medizinprodukt geeignet sind.

Bis(3,5-di-*tert*-butyl-2-hydroxyphenyl)selen (14), Bis(3-*tert*-butyl-5-ethyl-2-hydroxyphenyl)-selen (15), Bis(3,5-di(1,1-dimethylpropyl)-2-hydroxyphenyl)selen (16) oder Bis(5,6-dimethyl-2-hydroxy-3-isopropylphenyl)selen (18) können ggf. als Mikrobizide ohne fungizide Wirkung verwendet werden.

Ferner kann mindestens ein Selenodiaryl der Formel **I** zur Anwendung als Arzneimittel, insbesondere als mikrobizid wirkendes Arzneimittel, wie vorzugsweise als pharmazeutische fungizide Salbe, oder als Mikrobizid ausgewählt sein aus der allgemeinen Formel **Ib** 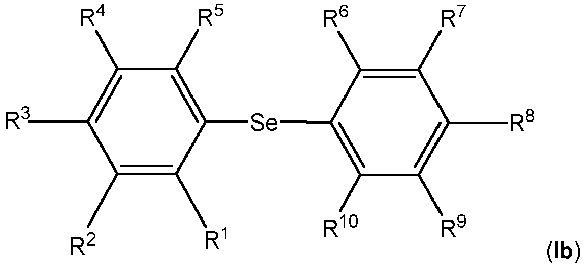 worin R¹, R², R³, R⁴, R⁵, R⁶, R⁷, R⁸, R⁹ und R¹⁰ jeweils unabhängig voneinander ausgewählt sind aus: -H, -OH, -Halogen, insbesondere -Fluor, -Chlor, -Brom, -(C₁-C₄)-Alkyl, -O-(C₁-C₄)-Alkyl, wobei die Alkylgruppen jeweils unabhängig unsubstituiert oder substituiert sind, wobei substituierte -(C₁-C₄)-Alkylgruppen mindestens einen Substituenten aufweisen der ausgewählt ist aus -Halogen.

Bevorzugte Selenodiaryle der Formel **Ib** umfassen Strukturen, in denen R¹, R², R³, R⁴, R⁵, R⁶, R¹, R⁸, R⁹ und R¹⁰ jeweils unabhängig voneinander ausgewählt sind aus: -H, -OH, -Halogen, insbesondere -Fluor, -Chlor, -Brom, -(C₁-C₃)-Alkyl, -O-(C₁-C₃)-Alkyl, wobei die Alkylgruppen jeweils unsubstituiert sind.

Besonders bevorzugte Selenodiaryle der Formel **Ib** umfassen Strukturen, in denen R¹, R², R³, R⁴, R⁵, R⁶, R⁷, R⁸, R⁹ und R¹⁰ jeweils unabhängig voneinander ausgewählt sind aus: -H, -OH, -Methyl, -OMethyl, wobei die Alkylgruppen jeweils unsubstituiert sind.

**Tabelle 3: Selenodiaryle der Formel Ib mit R¹ und R¹⁰ gleich -Methyl oder -OMethyl**

| **Produkt** | **Reste** | | | | |
|---|---|---|---|---|---|
| 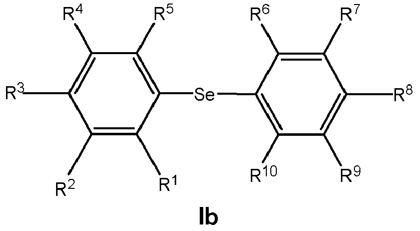 | R¹, R¹⁰ | R², R⁹ | R³,R⁸ | R⁴, R⁷ | R⁵ R⁶ |
| 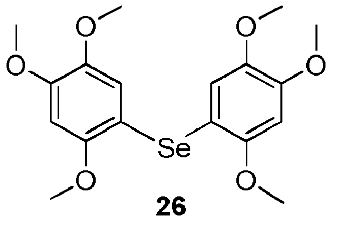 | -O-Me | -H | -O-Me | -O-Me | -H |
| 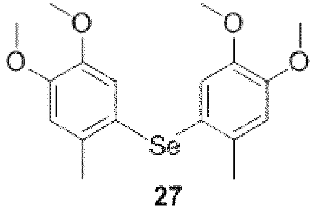 | -O-Me | -O-Me | -O-Me | -H | -Me |
| 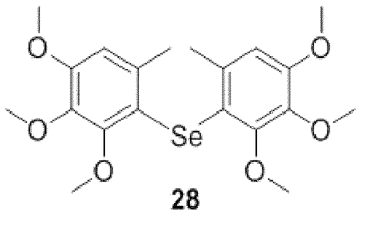 | -Me | -H | -O-Me | -O-Me | -H |

Gleichfalls bevorzugt ist Bis(2,4,5-trimethoxyphenyl)selen (26) der allgemeinen Formel **I,** da diese Verbindung keinen Schädigung von Nutzpflanzen verursacht.

Ebenfalls bevorzugt sind Bis(3,4-dimethoxy-6-methylphenyl)selen (27) und Bis(6-methyl-2,3,4-trimethoxyphenyl)selen (28), diese Verbindungen sind insbesondere für eine Anwendung als Biozid, Desinfektionsmittel, Werkstoffschutzmittel, Medizinprodukt geeignet.

Nach einer weiteren bevorzugten Ausführungsform kann mindestens ein Selenodiaryl der Formel **I** zur Anwendung als Arzneimittel verwendet werden, insbesondere als mikrobizid wirkendes Arzneimittel, wie als fungizid wirkende pharmazeutische Salbe, oder als Mikrobizid.

Das Selenodiaryl kann ausgewählt sein aus: a) Bis(3,5-dimethyl-4-hydroxyphenyl)selen (19), Bis(3-chlor-5-fluor-2-hydroxyphenyl)selen (24) und/oder Bis(3,5-difluoro-2-hydroxyphenyl)selen (25), oder
b1) Bis(3,5-dimethyl-2-hydroxyphenyl)selen (12), Bis(5-*tert*-butyl-2-hydroxy-3-methylphenyl)selen (13), Bis(2-hydroxy-3-methoxy-5-methylphenyl)selen (20), Bis(3-chlor-6-hydroxy-5-isopropyl-2-methylphenyl)selen (21), Bis(3-chlor-6-hydroxy-5-methylphenyl)selen (22), Bis(3-brom-2-hydroxy-5-methylphenyl)selen (23), Bis(2,4,5-trimethoxyphenyl)selen (26), oder
b2) Bis(2-hydroxy-3,5,6-trimethylphenyl)selen (17), Bis(3,4-dimethoxy-6-methylphenyl)selen (27), Bis(6-methyl-2,3,4-trimethoxyphenyl)selen (28).

Entsprechend der vorstehenden Klassifizierung zeigen die Selenodiaryle in a) eine besondere Eignung als mikrobizid wirkendes Arzneimittel, als Desinfektionsmittel oder Werkstoffschutzmittel, die Selenodiaryle in b1) als Mikrobizid ohne Schaden an Nutzpflanzen und b2) als Mikrobizid mit nur geringem Einfluss auf Nutzpflanzen. Entsprechend einer weiteren bevorzugten Ausführungsform kann mindestens ein Selenodiaryl der Formel **I** zur Anwendung als mikrobizid wirkendes Arzneimittel oder als Mikrobizid verwendet werden und kann wirksam sein gegenüber *C*. *albicans, M. miehei, P. notatum, P. variotii; B. cinerea, P. infestans, M. grisea CM, M. grisea H₂O*, wobei die mikrobizide Wirksamkeit vorzugsweise für Selenodiaryl-Verbindungen mit den folgenden Nummern nachgewiesen ist: C. *albicans:* Selenodiaryl-Verbindungen der Nr. 24, 25; *M. miehei:* Selenodiaryl-Verbindungen der Nr. 19, 24, 25, 26; *P. notatum:* Selenodiaryl-Verbindungen der Nr. 19, 24, 25; *P. variotii:* Selenodiaryl-Verbindungen der Nr. 19, 24, 25; *B. cinerea:* Selenodiaryl-Verbindungen der Nr. 19, 22, 24, 25; P. *infestans:* Selenodiaryl-Verbindungen der Nr. 12, 17, 19, 20, 22, 24, 25; M. *grisea CM:* Selenodiaryl-Verbindungen der Nr. 12, 13, 17, 19, 20, 21, 22, 23, 24, 25, 26, 27, 28, insbesondere Selenodiaryl-Verbindungen der Nr. 12, 13, 21, 22 und 23; *M. grisea H₂O:* Selenodiaryl-Verbindungen der Nr. 13, 19, 20, 21, 22, 23, 24, 25, 26, 27, 28, insbesondere Selenodiaryl-Verbindungen der Nr. 13 und 22.

Gleichfalls Gegenstand der Erfindung ist mindestens ein Selenodiaryl zur Verwendung als a) mikrobizides Arzneimittel, insbesondere als Antimykotikum, vorzugsweise zur Anwendung in einem Verfahren zur Prävention und/oder Behandlung von Pilzinfektionen, Mykosen, wie Kandidosen und/oder Pilzkrankheiten bei Lebewesen, vorzugsweise bei Mensch oder Tier, oder
b) Mikrobizid, insbesondere als Antimykotikum, vorzugsweise zur Anwendung in einem Verfahren zur Prävention und/oder Behandlung von Pilzinfektionen und/oder Pilzkrankheiten
bei Pflanzen. Vorzugsweise handelt es sich bei dem Selenodiaryl nach Formel **I** um Bis(3-chlor-6-hydroxy-5-methylphenyl)selen (22).

Besonders bevorzugt sind gemäß a) Selenodiaryle zur Anwendung in einem Verfahren zur Prävention und/oder Behandlung von humanpathogenen Pilzen. Vorzugsweise wird das Selenodiaryl nach Formel **I** ausgewählt aus Bis(3,5-dimethyl-4-hydroxyphenyl)selen (19), Di(3-chlor-5-fluor-2-hydroxyphenyl)selen (24), Bis(3,5-difluoro-2-hydroxyphenyl)selen (25) und/oder Bis(2,4,5-trimethoxyphenyl)selen (26).

Nach einer weiteren bevorzugten Ausführungsform kann das mindestens eine Selenodiaryl der Formel **I** dienen zur Prävention und/oder Bekämpfung eines mikrobiziden, insbesondere fungiziden, Befalls organischer Materialien, anorganischer Materialien oder Hybridmaterialien umfassend organische und anorganische Materialien, insbesondere zur Prävention und/oder Bekämpfung eines mikrobiziden Befalls, bevorzugt eines fungiziden Befalls, mineralischer Werkstoffe, organischer Werkstoffe, Baumaterialien, Textilien, Lacken, Metallen, Legierungen, Holz, natürlichen Polymeren, synthetischen Polymeren, (PVC, Siliconen), elektronischer Bauteile, Lebensmitteln, Futtermitteln. Dabei umfassen Werkstoffe u.a. Metalle, Nichtmetalle, wie Graphit und/oder Kohlenstoff, organische Werkstoffe, wie Holz, Pflanzenfasern und/oder Kunststoff, anorganische Werkstoffe, wie Keramik und/oder Glas und/oder Halbleiter, wie Silicium. Nutzpflanzen umfassen u.a. Getreidepflanzen, Reispflanzen, Knollenpflanzen, Rübenpflanzen, Gemüsepflanzen, Obst und ihre jeweiligen Samen und Fruchtorgane. Lebensmittel umfassen Mandeln, Nahrungsergänzungsmittel, Käse etc. Werkstoffe umfassen u.a. Dicht-, Isoliermaterialien, Dämmstoffe, Fugenmittel, Silicone. Nach einer weiteren bevorzugten Ausführungsform kann das mindestens eine Selenodiaryl der Formel **I** dienen zur Prävention und/oder Bekämpfung eines mikrobiziden, insbesondere fungiziden, Befalls organischer Materialien, wie Holz, Faser, Leder, Gummi, Kunststoffe, Mauerwerk, Flüssigkeiten in Kühl/Wassersystemen, Beschichtung in Kühl-/ Abwassersystemen oder Wasser führenden Systemen, Metallbearbeitung.

Die Herstellung der Selenodiaryl wird offenbart in EP2949646A1, DE102015203967.6, EP15168377.8, PCT/EP2016/054419 und PCT/EP2016/054420.

Die Begriffe Selenodiaryl, Selenodiphenol, Phenol und Olefin werden in dieser Anmeldung als Gattungsbegriff verwendet und umfassen jeweils somit auch substituierte Selenodiaryle, Selenodiphenole, Phenole und Olefine.

Die vorgenannten Definitionen für substituierte -(C₁-C₁₂)-Alkylgruppen und substituierte -(C₆-C₂₀)-Arylgruppen gelten für alle Gruppen in denen diese Alkyl- oder Arylgruppen enthalten sind, also insbesondere auch für die folgenden Gruppen: -O-(C₁-C₁₂)-Alkyl, -(C₆-C₂₀)-Aryl, -O-(C₆-C₂₀)-Aryl.

Ein oder mehrere Substituenten umfassen vorzugsweise 1 bis 10 Substituenten, insbesondere 1 bis 3. Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt um -(C₁-C₆)-Alkylgruppen. Beispiele für -(C₁-C₁₂)-Alkylgruppen sind, insbesondere Methyl-, Ethyl-, Propyl, Isopropyl-, n-Butyl-, iso-Butyl-, sec.-Butyl-, tert.-Butyl-, n-Pentyl-, 2-Pentyl-, 2-Methylbutyl-, 3-Methylbutyl-, 1,2-Dimethylpropyl-, 1,1-Dimethylpropyl-, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl. Besonders bevorzugte -(C₁-C₁₂)-Alkylgruppen der Formeln **I, Ia, Ib, II, III** und **IV** umfassen Methyl-, Ethyl-, Propyl-, Isopropyl-, n-Butyl-, *iso*-Butyl-, sec.-Butyl-, *tert*.-Butyl-, *tert*.-Pentyl- insbesondere Methyl-, Ethyl-, Propyl,- Isopropyl-.

Halogen (Hal) umfasst Chlor, Brom und Jod, wobei Chlor und Fluor besonders bevorzugt sind.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt aus -(C₃-C₁₂)-Cycloalkyl, -(C₃-C₁₂)-Heterocycloalkyl, -(C₆-C₂₀)-Aryl, -Fluor, -Chlor, -Cyano, -Formyl, -Acyl oder -Alkoxycarbonyl. Diese Definition gilt für alle substituierten Alkyl- oder Alkyoxygruppen der vorliegenden Erfindung.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.
Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl-. Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Hetero-cycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrahydrofuryl, Tetrahydropyranyl und Dioxanyl.

Ebenso ist Gegenstand der Erfindung eine Zusammensetzung umfassend mindestens ein Selenodiaryl der Formel **I**, insbesondere der Formeln **Ia, Ib, II, III** oder **IV** oder eines Gemisches umfassend mindestens zwei der vorgenannten Selenodiaryle, zur Anwendung als Mikrobizid (Antimykotikum) oder Arzneimittel, insbesondere als mikrobizides Arzneimittel, worin R¹, R², R³, R⁴, R⁵, R⁶, R⁷, R⁸, R⁹ und R¹⁰ jeweils unabhängig voneinander ausgewählt sind aus: -H, -OH, -(C₁-C₁₂)-Alkyl, -O-(C₁-C₁₂)-Alkyl, -(C₆-C₂₀)-Aryl, -O-(C₆-C₂₀)-Aryl, -Halogen, insbesondere -Fluor, -Chlor, -Brom oder -Jod, -S-Alkyl, -S-Aryl, -CO-(C₁-C₁₂)-Alkyl, -CO-(C₆-C₂₀)-Aryl, -(C₁-C₁₂)-Alkyl-O-(C₁-C₁₂)-Alkyl, -(C₁-C₁₂)-Alkyl-(C₆-C₂₀)-Aryl,-(C₁-C₁₂)-Alkyl-O-(C₆-C₂₀)-Aryl, -(C₆-C₂₀)-Aryl-O-(C₆-C₂₀)-Aryl, -(C₆-C₂₀)-Aryl-(C₁-C₁₂)-Alkyl, -(C₁-C₁₂)-Alkyl-O-(C₁-C₁₂)-Alkyl-(C₆-C₂₀)-Aryl, -(C₁-C₁₂)-Alkyl-(O-(C₁-C₁₂)-Alkyl)ₓ-O-(C₁-C₁₂)-Alkyl, mit x = 1 bis 3, -COOH, -SO₃H, -CN, wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, -Halogen, -Cyano, -Formyl, -Acyl oder -Alkoxycarbonyl.

Dabei ist es weiter bevorzugt, wenn die Zusammensetzung mindestens einen weiteren Wirkstoff, wie ein zweites Fungizid/Antimykotikum umfasst, optional in Gegenwart mindestens eines Hilfsstoffs.

Eine besonders bevorzugte Zusammensetzung kann vorliegen
i) in flüssiger Form umfassend Lösung, Injektion, Infusion, Tinktur, Tropflösung, Suspension, Emulsion, Dispersion, oder
ii) in fester Form umfassend Pulver, Lyophilisat, Tablette, Granulat, Filmtablette, Dragee, Kapsel, Brausetablette, Puder oder Seife, oder
iii) als Spray, Aerosol, Emulsion, Salbe, Paste und Kapsel, Gel, Schaum oder Creme.

Des Weiteren kann die Zusammensetzung vorliegen als eine Formulierung zur Anwendung als Arzneimittel, Kosmetikum, wie Shampoo, Medizinprodukt, Pflanzenschutzmittel, Desinfektionsmittel, Biozid, Werkstoffschutzmittel, Nahrungsergänzungsmittel, Futtermittel oder das Selenodiaryl der allgemeinen Formel liegt als Additiv zur Anwendung als Mikrobizid in einer Zusammensetzung vor.

Gleichfalls Gegenstand der Erfindung ist die Verwendung eines Selenodiaryls der allgemeinen Formel I zur Herstellung einer mikrobizid wirkenden nicht medizinischen Ausrüstung oder zur Herstellung einer mikrobizid wirkenden nicht medizinischen Zusammensetzung und/oder zur mikrobizid wirkenden Funktionalisierung oder Ausrüstung von funktionalisierbaren Substraten.

Ebenso Gegenstand der Erfindung ist eine fungistatische und/oder fungizide Verwendung der Selenodiaryle der allgemeinen Formel I, insbesondere nicht am menschlichen oder tierischen Körper. Eine bevorzugte fungistatische und/oder fungizide Verwendung der Selenodiaryle der allgemeinen Formel I kann umfassen eine Verwendung als Pflanzenschutzmittel, Desinfektionsmittel, Werkstoffschutzmittel, Additiv zur fungistatischen und/oder fungiziden Ausrüstung von Zusammensetzungen, Substraten in der Masse oder auf der Substraoberfläche.

Nachfolgend wird die Erfindung näher an Beispielen erläutert, ohne die Erfindung auf die Ausführungsbeispiele zu beschränken.

### Allgemeine Arbeitsvorschriften

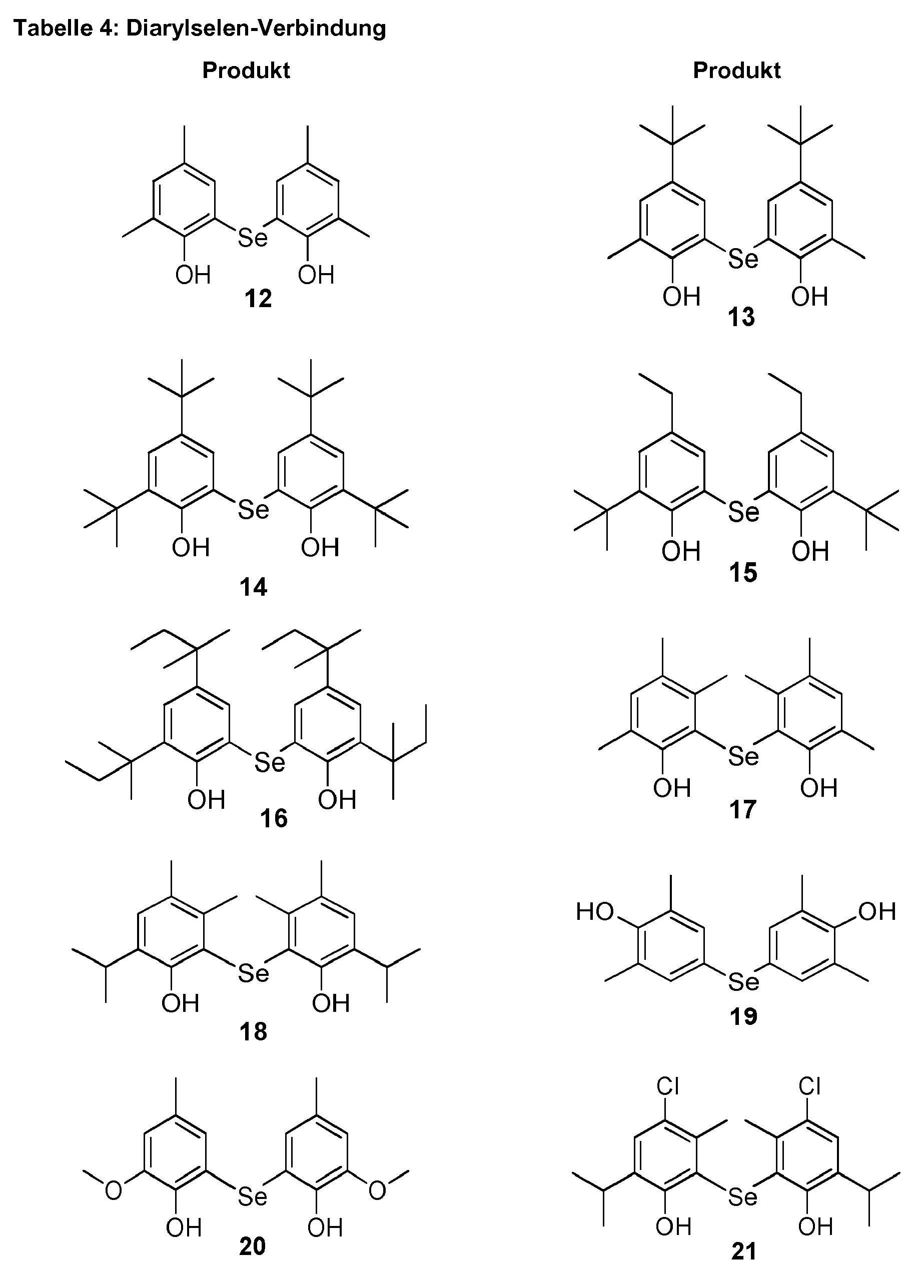

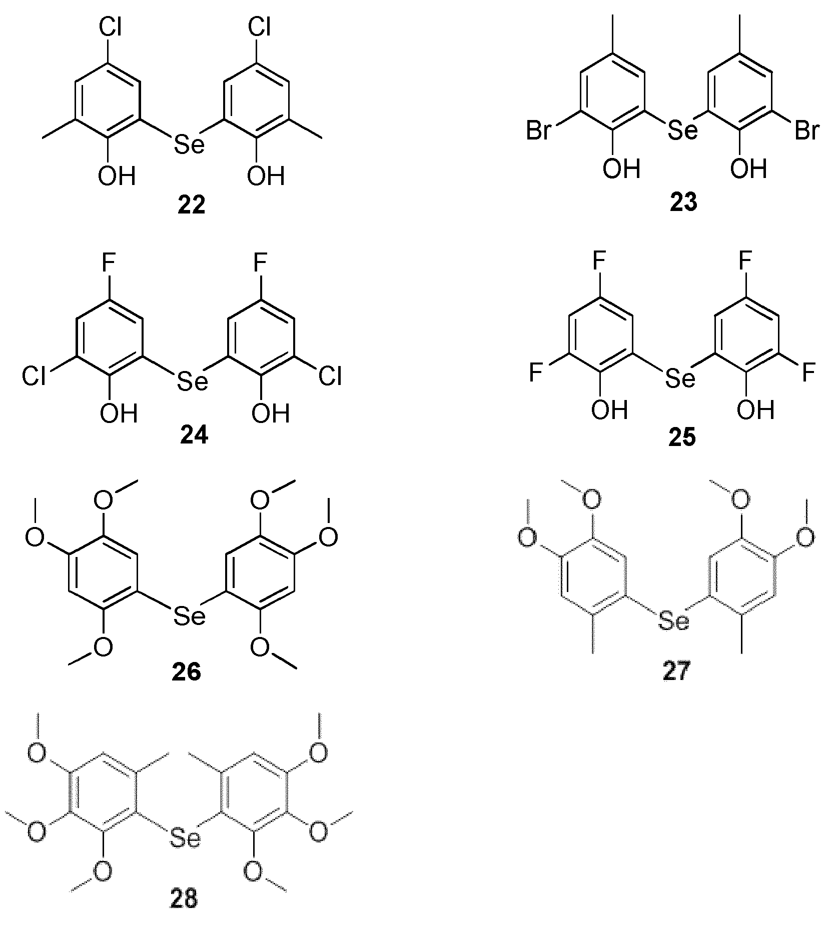

In der Tabelle 4 sind die hergestellten Diarylselen-Verbindungen dargestellt, die auf eine Aktivität gegen Pathogenen getestet wurden.

### Probenbereitung

Um die Aktivitäten der zu testenden Substanzen in Abhängigkeit von der Konzentration testen zu können wurden verschieden konzentrierte Lösungen erstellt (5 µg/mL, 10 µg/mL, 25 µg/mL, 50 µg/mL). Als Lösungsmittel wurde Dimethylsulfoxid verwendet.

Die Untersuchung der einzelnen Pathogene entspricht dem neuesten Stand der Technik und wurde wie in der Literatur beschrieben durchgeführt^{]}
(J. Yip, L. Liu, W. Liwei, K. Wong, P. Leung, H. M. Polly, C. M. Yuen, M. Cheung J. Nat. Prod. 2012, 75, 1405-1408).

Die Testung erfolgte wie folgt:

### Wachstumstests mit Candida albicans, Mucor miehei, Penicillium notatum und Paecilomyces variotii

Inokulierte HA-Agarplatten von ***Candida** albicans* (37 °C Inkubationstemperatur), *Paecilomyces variotii* (37 °C Inkubationstemperatur), *Penicillium notatum* (27 °C Inkubationstemperatur) und *Mucor miehei* (37 °C Inkubationstemperatur) werden zwei bis drei Tage vor Testbeginn bei entsprechender Temperatur inkubiert. Sporen der Kulturen von *Paecilomyces variotii, Penicillium notatum* und *Mucor miehei* werden von diesen mit HA-Flüssigmedium abgeschwemmt, auf eine Konzentration von etwa 1 x 10⁵ Sporen/mL eingestellt, in Schüttelkolben überführt und für weitere 3-4 h bei 120 rpm und der entsprechenden Inkubationstemperatur kultiviert. Für die Testung von *Candida albicans* werden einige Kolonien von der Platte abgenommen, in HA-Medium resuspendiert und eine Zelldichte von 1 x 10⁵ Zellen/mL eingestellt. In Mikrotiterplatten (96-well) werden die Testsubstanzen und Kontrollen vorgelegt, dann erfolgt die Inokulation mit den zuvor vorbereiteten Testorganismen. Die Mikrotiterplatten werden anschließend für 24 h bei der entsprechenden Inkubationstemperatur und 120 rpm inkubiert. Die Auswertung erfolgt optisch (+: kein Wachstum im Vergleich zur Wachstumskontrolle, -: Wachstum wie Wachstumskontrolle).
HA-Medium: 4 g/L Glucose, 4 g/L Hefeextrakt, 10 g/L Malzextrakt, pH 5,5 vor dem Autoklavieren (für Festmedien Zugabe von 2 % Agar)

### Keimungshemmungstests mit Magnaporthe grisea, Botrytis cinerea und Phytophthera infestans

Zur Gewinnung von Sporen werden die verschiedenen Pilze entsprechend ihrer Inkubationstemperatur, -dauer und -Medium angezogen (*Magnaporthe grisea* bei 27°C für 7-11 Tage auf CM- Agarplatten, *Botrytis cinerea* bei 22-24 °C für 3-4 Wochen auf 2 %igen Malzagar-Platten, *Phytophthora infestans* bei 18 °C für 2 bis 4 Wochen auf Erbsenmedium-Agarplatten). Für die Sporenpräparation werden die bewachsenen Agarplatten abgeschwemmt (*Magnaporthe grisea* und *Botrytis cinerea* mit sterilem Wasser und *Phytophthora infestans* mit Erbsenmedium), über Miracloth (Schnellfiltermaterial) filtriert, abzentrifugiert und in den entsprechenden Testmedien in definierter Konzentration aufgenommen (*Magnaporthe grisea* in Wasser und CM zu 1 x 10⁵ Sporen/mL, und *Botrytis cinerea* mit 2 %igen Malzmedium zu 1 x 10⁵ Sporen/mL und *Phytophthora infestans* in Erbsenmedium zu 2 x 10⁴ Sporen/mL). In Mikrotiterplatten (96-well) werden die Testsubstanzen und Kontrollen vorgelegt, dann erfolgt die Inokulation mit den zuvor vorbereiteten Testorganismen. Die Auswertung erfolgt nach 24 h Inkubation bei 22-24 °C mikroskopisch (+++: mehr als 75 % der Sporen nicht gekeimt, ++: zwischen 50 und 75 % der Sporen nicht gekeimt, +: zwischen 25 und 50 % der Sporen nicht gekeimt, -: zwischen 0 und 25 % der Sporen nicht gekeimt).

CM-Medium: 1 g/L Bacto Casamino Acids, 2 g/L, Bacto Pepton, 1g/L Bacto Yeast Extract, 10 g/L Glucose, 50 ml/L Nitratsalzlösung (20x; Zusammensetzung: 120 g/L NaNO₃, 10,4 g/L KCl, 10,4 g/L MgSO₄x7 *H₂O*, 30,4 g/L KH₂PO₄ in deionisiertem H₂O), 1 ml/L Spurenelementlösung (1000x; Zusammensetzung: 22 g/L ZnSO₄ x 7 H₂O, 11 g/L H₃BO₃, 5 g/L MnCl₂ x 4 *H₂O*, 5 g/L FeSO₄ x 7 *H₂O*, 1.7 g/L COCl₂ x 6 *H₂O*, 1.6 g/L CuSO₄ x 5 *H₂O*, 1.5 g/L Na₂MoO₄ x 2 *H₂O*, 50 g/L Na₂EDTA, pH 6,5 mit 1 M KOH eingestellt), pH 6,5 vor dem Autoklavieren (für Festmedien Zugabe von 2 % Agar)
2%iges Malzmedium: 20 g/L Malzextrakt, pH 5,5 vor dem Autoklavieren (für Festmedien Zugabe von 2 % Agar)
Erbsenmedium: 150 g/L Tiefkühlerbsen, 5 g/L Glucose, pH 6,5 vor dem Autoklavieren (für Festmedien Zugabe von 2 % Agar)

### Wachstumstests mit Setaria italica und Lepidium sativum

Testsubstanzen und Kontrollen werden auf Filterrondellen in Mikrotiterplatten (48 well) vorgelegt und das verwendete Lösungsmittel (DMSO) über Lyophilisation entfernt. Anschließend werden ca. 12 Samen pro Vertiefung (*Setaria italical Lepidium sativum*) zugeben, mit 200 µl sterilem Leitungswasser pro Vertiefung versetzt, mit einem Deckel versehen und in einer feuchten Kammer für 4 Tage im Pflanzenbrutschrank inkubiert (16h-Licht-8h-Dunkel-Rhythmus). Die Auswertung des Tests erfolgt optisch (+: mind. 75 % Wachstumshemmung im Vergleich zur Kontrolle, -: weniger als 75 % Wachstumshemmung im Vergleich zur Kontrolle.

In den Tabellen 5a bis 5d bedeutet: +: 50-75 % der Pilzkulturen wurden getötet; ++: 75-90 % der Pilzkulturen wurden getötet; +++: 100 % der Pilzkulturen wurden getötet.

### Testergebnisse

**Tabelle 5a: Aktivität der untersuchten Substanzen gegenüber Pilzen.**

| **Molekül** | ***C. albicans*** | | ***M. miehei*** | | ***P. notatum*** | | ***P. variotii*** | |
|---|---|---|---|---|---|---|---|---|
| | **10µg/mL** | **50µg/mL** | **10µg/mL** | **50µg/mL** | **10µg/mL** | **50µg/mL** | **10µg/mL** | **50µg/mL** |
| **12** | - | - | - | - | - | - | - | - |
| **13** | - | - | - | - | - | - | - | - |
| **14** | - | - | - | - | - | - | - | - |
| **15** | - | - | - | - | - | - | - | - |
| **16** | - | - | - | - | - | - | - | - |
| **17** | - | - | - | - | - | - | - | - |
| **18** | - | - | - | - | - | - | - | - |
| **19** | - | - | + | + | - | + | - | - |
| **20** | - | - | - | - | - | - | - | - |
| **21** | - | - | - | - | - | - | - | - |
| **22** | - | - | - | - | - | - | - | - |
| **23** | - | - | - | - | - | - | - | - |
| **24** | + | + | + | + | + | + | + | - |
| **25** | - | + | + | + | + | + | + | - |
| **26** | - | - | - | + | - | - | - | - |
| **27** | - | - | - | - | - | - | - | - |
| **28** | - | - | - | - | - | - | - | - |

**Tabelle 5b: Aktivität der untersuchten Substanzen gegenüber Pilzen.**

| **Molekül** | ***M. grisea H₂O*** | | | | ***B. cinerea*** | | | |
|---|---|---|---|---|---|---|---|---|
| | **5µg/mL** | **10µg/mL** | **25µg/mL** | **50µg/mL** | **5µg/mL** | **10µg/mL** | **25µg/mL** | **50µg/mL** |
| **12** | - | - | - | - | - | - | - | - |
| **13** | +++ | +++ | +++ | +++ | - | - | - | - |
| **14** | - | - | - | - | - | - | - | - |
| **15** | - | - | - | - | - | - | - | - |
| **16** | - | - | - | - | - | - | - | - |
| **17** | - | - | - | - | - | - | - | - |
| **18** | - | - | - | - | - | - | - | - |
| **19** | +++ | +++ | +++ | +++ | + | +++ | +++ | - |
| **20** | + | +++ | +++ | +++ | - | - | - | - |
| **21** | + | +++ | +++ | +++ | - | - | - | - |
| **22** | +++ | +++ | +++ | +++ | +++ | +++ | +++ | +++ |
| **23** | - | + | +++ | +++ | - | - | - | - |
| **24** | +++ | +++ | +++ | +++ | +++ | +++ | +++ | +++ |
| **25** | +++ | +++ | +++ | +++ | + | +++ | +++ | +++ |
| **26** | + | +++ | +++ | +++ | - | - | - | - |
| **27** | - | - | - | + | - | - | - | - |
| **28** | +++ | +++ | +++ | +++ | - | - | - | - |

**Tabelle 5c: Aktivität der untersuchten Substanzen gegenüber Pilzen.**

| **Molekül** | ***P. infestans*** | | | | ***M. grisea CM*** | | | |
|---|---|---|---|---|---|---|---|---|
| | **5µg/mL** | **10µg/mL** | **25µg/mL** | **50µg/mL** | **5µg/mL** | **10µg/mL** | **25µg/mL** | **50µg/mL** |
| **12** | - | +++ | +++ | +++ | +++ | +++ | +++ | +++ |
| **13** | - | - | - | - | +++ | +++ | +++ | +++ |
| **14** | - | - | - | - | - | - | - | - |
| **15** | - | - | - | - | - | - | - | - |
| **16** | - | - | - | - | - | - | - | - |
| **17** | - | + | + | + | - | - | ++ | ++ |
| **18** | - | - | - | - | - | - | - | - |
| **19** | - | ++ | ++ | - | +++ | +++ | +++ | +++ |
| **20** | - | - | +++ | +++ | + | +++ | +++ | +++ |
| **21** | - | - | - | - | ++ | +++ | +++ | +++ |
| **22** | - | +++ | +++ | +++ | +++ | +++ | +++ | +++ |
| **23** | - | - | - | - | +++ | +++ | +++ | +++ |
| **24** | +++ | +++ | +++ | +++ | +++ | +++ | +++ | +++ |
| **25** | +++ | +++ | +++ | +++ | +++ | +++ | +++ | +++ |
| **26** | - | - | - | - | + | +++ | +++ | +++ |
| **27** | - | - | - | - | - | - | + | + |
| **28** | - | - | - | - | +++ | +++ | +++ | +++ |

**Tabelle 5d: Aktivität der untersuchten Substanzen gegenüber Nutzpflanzen.**

| **Molekül** | ***S. italica*** | | | **Kontrolle** | ***L. sativum*** | | | **Kontrolle** |
|---|---|---|---|---|---|---|---|---|
| | **10µg/R** | **20µg/R** | **50µg/R** | **H₂O** | **10µg/R** | **20µg/R** | **50µg/R** | **H₂O** |
| **12** | - | - | - | - | - | - | - | - |
| **13** | - | - | - | - | - | - | - | - |
| **14** | - | - | - | - | - | - | - | - |
| **15** | - | - | - | - | - | - | - | - |
| **16** | - | - | - | - | - | - | - | - |
| **17** | - | - | + | - | - | - | - | - |
| **18** | - | - | - | - | - | - | - | - |
| **19** | + | + | + | - | - | - | - | - |
| **20** | - | - | - | - | - | - | - | - |
| **21** | - | - | - | - | - | - | - | - |
| **22** | - | - | - | - | - | - | - | - |
| **23** | - | - | - | - | - | - | - | - |
| **24** | - | + | - | - | - | + | - | - |
| **25** | - | + | - | - | - | + | - | - |
| **26** | - | - | - | - | - | - | - | - |
| **27** | + | - | + | - | - | - | - | - |
| **28** | - | - | + | - | - | - | - | - |

In Tabelle 5a bis 5d sind die Aktivitäten der in Tabelle 4 abgebildeten Substanzen aufgelistet. Bei, C. *albicans, M. miehei, P. notatum, P. variotii, M. grisea, B. cinerea* und *P. infestans* handelt es sich um Pilze. Genaue Details zu den jeweiligen Pathogenen finden sich in Tabelle 6. S. *italica* und *L. sativum* sind Nutzpflanzen, die zur Kontrolle getestet wurden um unerwünschte Aktivitäten gegen Kulturpflanzen zu testen.

**Tabelle 6: Erläuterungen zu den untersuchten Pathogenen**

| **Abkürzung** | **Vollständiger Name** | **Bedeutung** |
|---|---|---|
| *C*. *albicans* | *Candida albicans* | Hefepilz, der auf und im menschlichen Organismus vorkommt |
| *M. miehei* | *Mucor miehei* | weit verbreiteter Schimmelpilz |
| *P. notatum* | *Penicillium notatum* | Schimmelpilz, wächst verbreitet auf verdorbenen Nahrungsmitteln |
| *P. variotii* | *Paecilomyces variotii* | weit verbreiteter Schimmelpilz; bedeutender Erreger von Pilzinfektionen beim Menschen |
| *M. grisea* (H₂O) | *Magnaporthe grisea* | phytopathogener Pilz, verursacht gefährliche Erkrankungen bei Reispflanzen in der Keimphase |
| *B. cinerea* | *Botrytis cinerea* | verursacht Grauschimmel und Fäulnis bei vielen Kulturpflanzen |
| *P. infestans* | *Phytophthera infestans* | Erreger der Kraut- und Knollenfäule bei Kartoffelpflanzen |
| *M. grisea* (CM) | *Magnaporthe grisea* | Pilz der besonders Reispflanzen während der Wachstumsphase befällt |
| S. *italica* | *Setaria italica* | Kolbenhirse, vorwiegend in Asien angebaute Kulturpflanze |
| *L. sativum* | *Lepidium sativum* | Gartenkresse, Kulturpflanze |

Der Wachstumstest mit C. *albicans* zeigte eine deutliche Aktivität der Verbindungen **24** und **25.** Der Wachstumstest mit M. *miehei, P. notatum* und *P. variotii* zeigte darüber hinaus eine deutliche Aktivität der Verbindungen **19, 24** und **25,** weshalb die Verbindungen **19, 24** und **25** als Gruppe a) zusammengefasst werden. Im Keimungshemmungstest der Gruppe mit *M*. *grisea, B. cinerea* und *P. infestans* dagegen besaßen nur die Verbindungen **12, 13, 17, 20, 21, 22, 23, 26, 27** und **28,** entsprechend zusammengefasst als Gruppe b), eine fungizide Wirkung in Bezug auf die zugehörigen Pathogene. Wobei gegenüber *P. infestans* Verbindungen 13, 21, 23, 26, 27 und 28 keine Wirkung zeigte. Eine gute Wirksamkeit gegenüber *P. infestans* weisen die Verbindungen **12, 17, 19, 20, 22, 24** und **25** auf. Die Verbindungen **14, 15, 16** und **18** zeigten weder im Wachstums- noch im Keimungshemmungstest im verwendeten Konzentrationsbereich eine entsprechende Aktivität. Der zur Kontrolle durchgeführte Wachstumstest mit S. *italica* und *L. sativum* zeigte einen wachstumshemmenden Effekt der Verbindungen **19, 24** und **25** aus Gruppe a) sowie der Verbindungen **17, 27** und **28** aus Gruppe b) auf mindestens eine der beiden Nutzpflanzen. Aufgrund des unterschiedlichen Effekts der Mitglieder der Gruppe b) auf die beiden Nutzpflanzen, werden die Verbindungen **17, 27** und **28** in der Untergruppe b2) gruppiert, während die restlichen Verbindungen dementsprechend als Gruppe b1) zusammengefasst werden. Gegenüber den beiden Nutzpflanzen zeigten die *ortho*verknüpften, alkylierten Derivate auch in hohen Konzentrationen keine Aktivitäten.

Im Bereich der alkylsubstituierten Bis(hydroxyphenyl)selen-Verbindungen zeigten besonders die Derivate **12** und **13** selbst bei geringen Konzentrationen eine hohe Aktivität gegenüber *P. infestans* und *M. grisea,* ohne dabei einen negativen Effekt auf die getesteten Nutzpflanzen auszuüben. Verbindung **19** dagegen zeigte zwar eine deutliche Aktivität gegenüber allen getesteten Pathogenen, mit Ausnahme von *C*. *albicans,* übte aber auch einen wachstumshemmenden Effekt auf S. *italica* aus. Unter den Methoxy-substituierten Bis(hydroxyphenyl)selen-Verbindungen konnte nur für die Derivate **20** und **26** ein fungizider Effekt ohne gleichzeitige Nutzpflanzen-Schädigung nachgewiesen werden. Zudem zeigten die Verbindungen **26** bis **28** eine selektive fungizide Wirkung gegen *M. grisea.* Die halogenierten Bis(hydroxyphenyl)selen-Derivate **21** bis **25** besaßen auch in geringsten Konzentrationen hohe Aktivitäten, wobei die Verbindungen **20** bis **23** eine starke fungizide Wirkung im Keimungshemmungstest und gleichzeitig keine Schädigung in Bezug auf die getesteten Nutzpflanzen zeigten. Dagegen stachen die Derivate **24** und **25** durch durchgehende Aktivität gegen alle Pathogene im Keimungs- und Wachstumstest hervor, zeigten aber euch einen deutlichen negativen Effekt auf S. *italica* und *L. sativum.* Die getesteten Diarylselenide **26** bis **28** zeigten eine selektive fungizide Wirkung gegen *M. grisea,* in hohen Konzentrationen jedoch 27 und 28 auch eine Aktivität gegen Nutzpflanzen.

### Herstellung

### Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma *Macherey-Nagel GmbH* & *Co*, Düren durchgeführt. Die Trennungen ohne Druckbeaufschlagung wurden an Kieselgel Geduran Si 60 (0.063-0.200 mm) der Firma *Merck KGaA,* Darmstadt durchgeführt. Die als Eluentien verwendeten Lösungsmittel (Essigsäureethylester (technisch), Cyclohexan (technisch)) wurden zuvor destillativ am Rotationsverdampfer gereinigt.

Zur Dünnschichtchromatographie (DC) wurden PSC-Fertigplatten Kieselgel 60 F254 der Firma *Merck KGaA,* Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3 g konzentrierte Schwefelsäure auf 200 mL Wasser.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma *Shimadzu,* Japan. Es wird an einer Quarzkapillarsäule HP-5 der Firma *Agilent Technologies,* USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma *Shimadzu,* Japan aufgenommen. Es wird an einer Quarzkapillarsäule HP-1 der Firma *Agilent Technologies,* USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der lonenquelle: 200 °C) gemessen.

### Schmelzpunkte

Schmelzpunkte wurden mit Hilfe des Schmelzpunktbestimmungsgerätes SG 2000 der Firma *HW5,* Mainz gemessen und sind unkorrigiert.

### Massenspektrometrie

Alle Elektrosprayionisation-Messungen (ESI+) wurden an einem QTof Ultima 3 der Firma *Waters Micromasses,* Milford, Massachusetts durchgeführt. EI-Massenspektren sowie die hochaufgelösten EI-Spektren wurden an einem Gerät des Typs MAT 95 XL Sektorfeldgerät der Firma *Thermo Finnigan,* Bremen, gemessen.

### NMR-Spektroskopie

Die NMR-spektroskopischen Untersuchungen wurden an Multikernresonanzspektrometern des Typs AC 300 oder AV II 400 der Firma *Bruker,* Analytische Messtechnik, Karlsruhe, durchgeführt. Als Lösungsmittel wurde CDCI3 verwendet. Die ¹H- und ¹³C-Spektren wurden gemäß dem Restgehalt an nicht deuteriertem Lösungsmittel nach der *NMR Solvent Data Chart* der Fa. *Cambridge Isotopes Laboratories,* USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschrift AAV1 zur Darstellung von Diarylseleniden AAV1.

Die entsprechenden Phenole (1 Äquivalent) wurden zu einer Mischung aus Selendioxid (0.6 Äquivalente) in Pyridin gegeben und für 2-18 Stunden bei 55-85 °C gerührt. Anschließend wurden die Reaktionsmischungen mit Essigsäureethylester verdünnt, filtriert und die organische Phase mit Salzsäure (10%) und Wasser gewaschen. Nach Abtrennung der organischen Phase wurde diese über Magnesiumsulfat getrocknet und das Lösungsmittel unter vermindertem Druck abdestilliert. Das Rohprodukt wurde jeweils säulenchromatographisch gereinigt.

### Allgemeine Arbeitsvorschrift AAV1 zur Darstellung von Diarylseleniden AAV2.

Die entsprechenden Phenole (1 Äquivalent) wurden in Essigsäure gelöst, mit Selendioxid (0.6 Äquivalente) versetzt und bei 85 °C 2-12 Stunden gerührt. Anschließend wurden die Reaktionsmischungen mit Essigsäureethylester verdünnt, filtriert und die organische Phasen mit Kochsalzlösung gewaschen. Nach Abtrennung der organischen Phase wurde diese über Magnesiumsulfat getrocknet und das Lösungsmittel unter vermindertem Druck abdestilliert. Das Rohprodukt wurde jeweils säulenchromatographisch gereinigt.

### Bis(3,5-dimethyl-2-hydroxyphenyl)selen (12)

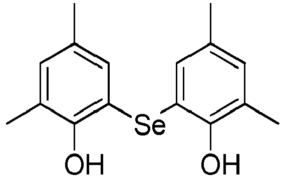

In einem 250 mL Rundkolben wurden 49.9 g Selendioxid (413 mmol) in 100 mL Pyridin mit Hilfe eines Ölbades auf 55 °C erwärmt. Anschließen wurden 25 mL 2,4-Dimethylphenol (206 mmol) hinzugegeben und die Temperatur für siebeneinhalb Stunden gehalten. Nach Beendigung der Reaktion wurde das Gemisch mit 400 mL Ethylacetat verdünnt und filtriert. Die organische Phase wurde mit Wasser gewaschen und über Magnesiumsulfat getrocknet. Das Pyridin wurde destillativ entfernt und der Rückstand erneut in Ethylacetat gelöst und mit 10 %iger Salzsäure und Wasser gewaschen um Reste von Pyridin zu entfernen. Die organische Phase wurde mit Magnesiumsulfat getrocknet und unter vermindertem Druck vom Lösungsmittel befreit. Das so erhaltene Rohprodukt wurde in 400 mL Cyclohexan unter Rückfluss erhitzt. Nach Abkühlung auf Raumtemperatur kristallisierte das Produkt. Nach einem Tag wurde das Produkt abfiltriert, das Filtrat auf die Hälfte eingeengt und bei 4 °C erneut zur Kristallisation gebracht. Es wurden 18.56 g, 58 mmol (56%) feine, leicht gelbe Platten des Produktes erhalten

mₚ= 120.1 °C (Rekristallisation aus Cyclohexan); ¹H NMR (400 MHz, CDCl₃) δ= 7.11-7.12 (m, 2H), 6.90-6.92 (m, 2H), 5.95 (br, 2H, OH), 2.23 (s, 6H), 2.19 (s, 6H); ¹³C NMR (100 MHz, CDCl₃) δ= 152.04, 133.35, 133.30, 130.67, 124.42, 115.31, 20.45, 16.69; ⁷⁷Se NMR (76 MHz, CDCl₃) δ= 164.91; HRMS für C₁₆H₁₈O₂⁸⁰Se (ESI+) [M+Na⁺]: berechnet: 345,0370, gefunden: 445.0363; Elementaranalyse für C₁₆H₁₈O₂Se: berechnet: C: 59.82%, H: 5.65%, gefunden: C: 59.69%, H: 5.76%.

### Di(5-tert-butyl-2-hydroxy-3-methylphenyl)selen (13)

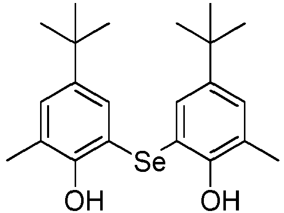

Wie in AAV1 beschrieben wurden 0.80 g 4-*tert*-Butyl-2-methylphenol (4.9 mmol, 1.0 eq) zu einer Lösung von 0.33 g Selendioxid (2.9 mmol, 0.6 eq.) in 6.7 mL Pyridin gegeben und bei 55 °C für 56 Stunden gerührt. Die Reaktionsmischung wurde mit 50 mL Ethylacetat verdünnt, filtriert und dreimal mit je 50 mL Salzsäure (10%) und einmal mit 50 mL Kochsalzlösung gewaschen. Nach dem Trocknen über Magnesiumsulfat wurde das Lösungsmittel unter vermindertem Druck entfernt und der erhaltene Rückstand säulenchromatographisch gereinigt (Laufmittel Cyclohexan/ Ethylacetat 99:1). Ausbeute: 36%, 0.35 g, 0.9 mmol.

mₚ= 98.5 °C; ¹H NMR (400 MHz, CDCl₃) δ= 7.30 (d, ⁴*J*=2.4 Hz, 2H), 7.11 (d, ⁴*J*=2.4 Hz, 2H), 5.92 (s, 2H, OH), 2.26 (d, 6H), 1.23 (s, 18H); ¹³C NMR (100 MHz, CDCl₃) δ= 151.78, 144.03, 129.83, 129.51, 123.89, 114.95, 34.18, 31.56, 16.99; HRMS für C₂₂H₃₀O₂⁸⁰Se (ESI+) [M+Na⁺]:berechnet: 429,1309, gefunden: 429.1250.

### Bis(3,5-di-tert-butyl-2-hydroxyphenyl)selen (14)

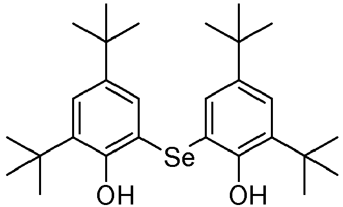

Wie in AAV1 beschrieben wurden 0.80 g 2,4-Di-*tert*-butylphenol (3.8 mmol, 1.0 eq.) zu einer Lösung von 0.25 g Selendioxid (2.3 mmol, 0.6 eq.) in 5.4 mL Pyridin gegeben und bei 55 °C für 4 Tage gerührt. Die Reaktionsmischung wurde mit 50 mL Ethylacetat verdünnt, filtriert und dreimal mit je 50 mL Salzsäure (10%) und einmal mit 50 mL Kochsalzlösung gewaschen. Nach dem Trocknen über Magnesiumsulfat wurde das Lösungsmittel unter vermindertem Druck entfernt und der erhaltene Rückstand säulenchromatographisch gereinigt (Laufmittel Cyclohexan/ Ethylacetat 99:1). Das gewünschte Produkt wurde bei 4 °C aus n-Heptan kristallisiert. Ausbeute: 25%, 0.24 g, 0.5 mmol.

mₚ= 141.1 °C (Rekristallisation aus Heptan); ¹H NMR (400 MHz, CDCl₃) δ= 7.31 (d, ⁴*J*=2.4 Hz, 2H), 7.29 (d, ⁴*J*=2.4 Hz, 2H), 6.29 (s, 2H, OH), 1.42 (s, 18H), 1.24 (s, 18H); ¹³C NMR (100 MHz, CDCl₃) δ= 151.7, 143.5, 135.8, 129.8, 125.6, 117.2, 35.4, 34.4, 31.6, 29.7. HRMS für C₂₈H₄₂O₂⁸⁰Se (ESI+) [M+Na⁺]: berechnet: 513.2248, gefunden: 513.2152.

### Bis(3-tert-butyl-5-ethyl-2-hydroxyphenyl)selen (15)

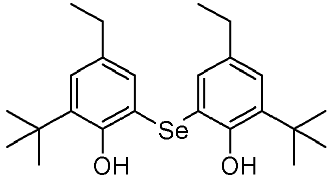

Wie in AAV1 beschrieben wurden 2.00 g 2-*tert*-Butyl-4-ethylphenol (15.8 mmol, 1.0 eq.) zu einer Lösung von 1.06 g Selendioxid (9.5 mmol, 0.6 eq.) in 18 mL Pyridin gegeben und bei 60 °C für 4 Tage gerührt. Die Reaktionsmischung wurde mit 50 mL Ethylacetat verdünnt, filtriert und dreimal mit je 50 mL Salzsäure (10%) und einmal mit 50 mL Kochsalzlösung gewaschen. Nach dem Trockenen über Magnesiumsulfat wurde das Lösungsmittel unter vermindertem Druck entfernt und der erhaltene Rückstand säulenchromatographisch gereinigt (Laufmittel: Cyclohexan/ Ethylacetat 99:1). Ausbeute: 27%, 0.659 g, 1.5 mmol.
mₚ= 68.2 °C; ¹H NMR (400 MHz, CDCl₃) δ= 7.18 (d, ⁴*J*= 2.1 Hz, 2H), 7.07 (d, ⁴*J*= 2.1 Hz, 2H), 6.32 (s, 2H, OH), 2.51 (q, ³*J*= 7.6 Hz, 4H), 1.40 (s, 18H), 1.16 (t, ³*J*= 7.6 Hz, 6H).

¹³C NMR (100 MHz, CDCl₃) δ= 152.00, 136.42, 136.32, 131.98, 128.22, 117.19, 35.09, 29.53, 28.12, 15.66; HRMS für C₂₄H₂₄O₂⁸⁰Se (ESI+) [M+Na⁺]: berechnet: 457.1622, gefunden: 457.1632; Elementaranalyse für C₂₄H₂₄O₂Se: berechnet: C: 66.50%, H: 7.38%, gefunden: C: 66.26%, H: 7.54%.

### Bis(3,5-di(1,1-dimethylpropyl)-2-hydroxyphenyl)selen (16)

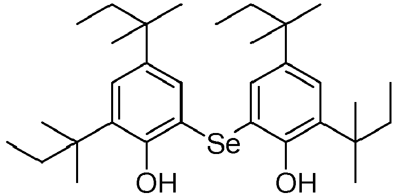

Wie in AAV1 beschrieben wurden 2.00 g 2,4-Di-(1,1-dimethylpropyl)phenol (13.6 mmol, 1.0 eq.) zu einer Lösung von 0.91 g Selendioxid (8.2 mmol, 0.6 eq.) in 19 mL Pyridin gegeben und bei 60 °C für 4 Tage gerührt. Die Reaktionsmischung wurde mit 50 mL Ethylacetat verdünnt, filtriert und dreimal mit je 50 mL Salzsäure (10%) und einmal mit 50 mL Kochsalzlösung gewaschen. Nach dem Trocknen über Magnesiumsulfat wurde das Lösungsmittel unter vermindertem Druck entfernt und der erhaltene Rückstand säulenchromatographisch gereinigt (Laufmittel: Cyclohexan/ Ethylacetat mit einem Gradienten von 100:0 bis 95:5). Ausbeute: 25%, 0,586 g, 1.1 mmol.

mₚ=119.7 °C; ¹H NMR (400 MHz, CDCl₃) δ= 7.18 (d, ⁴*J*= 2.3 Hz, 2H), 7.11 (d, ⁴*J*= 2.3 Hz, 2H), 6.15 (s, 2H), 1.82 (q, ³*J*= 7.5 Hz, 4 H), 1.50 (q, ³*J*= 7.4 Hz, 4 H), 1.34 (s, 12H), 1.16 (s, 12H), 0.58 (q, ³*J*= 7.5 Hz, 12 H); ¹³C NMR (100 MHz, CDCl₃) δ= 151.34, 142.32, 133.78, 130.15, 127.34, 116.99, 38.85, 37.45, 36.94, 33.11, 28.45, 27.72, 9.46, 9.01.
HRMS for C₂₂H₃₀O₂⁸⁰Se (ESI+) [M+Na⁺]: berechnet: 569.2874, gefunden: 569.2800.

### Bis(2-hydroxy-3,5,6-trimethylphenyl)selen (17)

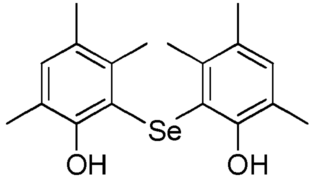

Wie in AAV1 beschrieben wurden 0.70 g 2,4,5-Trimethylphenol (5.1 mmol, 1.0 eq.) zu einer Lösung von 0.34 g Selendioxid (3.0 mmol, 0.6 eq.) in 7.1 mL Pyridin gegeben und bei 60 °C für 30 Stunden gerührt. Die Reaktionsmischung wurde mit 50 mL Ethylacetat verdünnt, filtriert und dreimal mit je 50 mL Salzsäure (10%) und einmal mit 50 mL Kochsalzlösung gewaschen. Nach dem Trocknen über Magnesiumsulfat wurde das Lösungsmittel unter vermindertem Druck entfernt und der erhaltene Rückstand säulenchromatographisch gereinigt (Laufmittel: Cyclohexan/ Ethylacetat 500:1). Das so erhaltene Produkt wurde bei 4 °C aus Cyclohexan kristallisiert. Ausbeute: 36%, 0.33 g, 0.9 mmol.

mₚ= 123.1 °C (Rekristallisation aus Cyclohexan); ¹H NMR (400 MHz, CDCl₃) δ= 6.90 (s, 2H), 6.18 (s, 2H, OH), 2.36 (s, 6H), 2.19 (s, 6H), 2.18 (s, 6H); ¹³C NMR (100 MHz, CDCl₃) δ= 152.80, 137.68, 133.35, 128.66, 121.45, 116.44, 20.39, 19.79, 16.39; HRMS für C₁₈H₂₂O₂⁸⁰Se (ESI+) [M+Na⁺]: berechnet: 373.0683, gefunden: 373.0693; Elementaranalyse C₁₈H₂₂O₂Se: berechnet: C: 61.89%, H: 6.35%, gefunden: C: 61.77.41%, H: 6.34%.

### Bis(5,6-dimethyl-2-hydroxy-3-isopropylphenyl)selen (18)

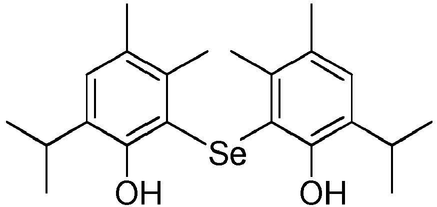

Wie in AAV1 beschrieben wurden 0.80 g 4,5-Dimethyl-2-isopropylphenol (4.9 mmol, 1.0 eq.) zu einer Lösung aus 0.32 g Selendioxid (2.9 mmol, 0.6 eq.) in 7.5 mL Pyridin gegeben und bei 60 °C für 70 Stunden gerührt. Die Reaktionsmischung wurde mit 50 mL Ethylacetat verdünnt, filtriert und dreimal mit je 50 mL Salzsäure (10%) und einmal mit 50 mL Kochsalzlösung gewaschen. Nach dem Trocknen über Magnesiumsulfat wurde das Lösungsmittel unter vermindertem Druck entfernt und der erhaltene Rückstand säulenchromatographisch gereinigt (Laufmittel: Cyclohexan/ Ethylacetat 99:1). Das so erhaltene Produkt wurde bei 4 °C aus Acetonitril kristallisiert. Ausbeute: 39%, 0.38 g, 0.9 mmol.

mₚ= 125.6 °C (Rekristallsiation aus Acetonitril); ¹H NMR (400 MHz, CDCl₃) δ= 6.96 (s, 2H), 6.32 (s, 2H, OH), 3.22 (hept, ³*J*=6.9 Hz, 2H), 2.31 (s, 6H), 2.20 (s, 6H), 1.20 (d, ³*J*=6.9 Hz, 12H); ¹³C NMR (100 MHz, CDCl₃) δ= 151.99, 137.55, 132.15, 128.97, 116.92, 27.70, 22.69, 20.69, 20.87, 19.82; HRMS für C₂₂H₃₀O₂⁸⁰Se (ESI+) [M+Na⁺]: berechnet: 429.1309, gefunden: 429.1305; Elementaranalyse für C₂₂H₃₀O₂Se: berechnet: C: 65.17%, H: 7.06%, gefunden: C: 64.87%, H: 6.61%.

### Bis(3,5-dimethyl-4-hydroxyphenyl)selen (19)

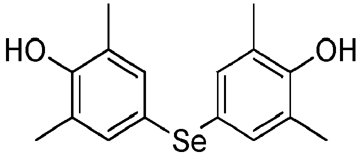

In einem Kulturröhrchen wurden 500 mg (4.1 mmol) 2,6-Dimethylphenol in 5.7 mL Pyridin gelöst und mit 250 mg Selendioxid (2.2 mmol) versetzt. Das Gemisch wurde in einem 55 °C heißen Stahlblock erhitzt. Nach 24 Stunden wurde das Reaktionsgemisch mit 40 mL Dichlormethan verdünnt und filtriert. Das Filtrat wurde zweimal mit je 30 mL Salzsäure (10%) und zweimal mit je 30 mL Wasser gewaschen. Die organische Phase wurde abgetrennt, mit Magnesiumsulfat getrocknet und vom Lösungsmittel befreit. Das so erhalten Rohprodukt wurde mittels Säulenchromatographie gereinigt. Dabei wurde ein Säulenautomat der Firma *BÜCHI-Labortechnik GmbH,* Essen verwendet. Die Säulenlänge betrug 16 cm und der Durchmesser 6 cm. Als Eluent wurde Cyclohexan/Essigsäureethylester verwendet, wobei mit einem Essigsäureethylester Gradienten gearbeitet wurde: 5-10 % (über 20 min), 25-100 %

(über 5 min). Das so erhaltene Produkt wurde in 20 mL Cyclohexan in der Siedehitze gelöst. Nach Abkühlung auf Raumtemperatur bildeten sich gelbliche Nadeln. Ausbeute: 53%, 7.01 g, 21.8 mmol.

mₚ= 220.7 °C (Rekristallisation aus Heptan); ¹H NMR (400 MHz, CDCl₃) δ=7.15 (s, 4H), 4.62 (s, 2H, OH), 2.21 (s, 12H); ¹³C NMR (100 MHz, CDCl₃) δ= 152.03, 133.71, 124.21, 121.48, 15.92; HRMS für C₁₆H₁₈O₂⁸⁰Se (ESI+) [M⁺]: berechnet: 322.0472, gefunden: 322.0457; Elementaranalyse für C₂₄H₂₄O₂Se: berechnet: C: 59.82%, H: 5.65%, gefunden: C: 59.41%, H: 5.30%.

### Bis(2-hydroxy-3-methoxy-5-methylphenyl)selen (20)

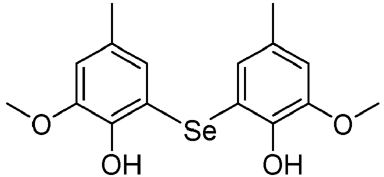

In einem 10 mL Rundkolben wurden 700 mg 4-Methylguaiacol (51 mmol) in 7.1 mL Pyridin gelöst, mit 0.856 g Selendioxid (77 mmol) versetzt und im Ölbad auf 55 °C erhitzt. Nach vier Tagen wurde die Reaktionslösung mit 50 mL Ethylacetat verdünnt und filtriert. Die organische Phase wurde zunächst zwei Mal mit je 40 mL 10 %iger Salzsäure und zwei Mal mit je 40 mL Wasser gewaschen. Nach dem Trocknen über Magnesiumsulfat wurde das Lösungsmittel destillativ entfernt und das Rohprodukt mittels Säulenchromatographie gereinigt. Dabei wurde ein Säulenautomat der Firma *BÜCHI-Labortechnik GmbH,* Essen verwendet. Die Säulenlänge betrug 16 cm und der Durchmesser 6 cm. Als Eluent wurde Cyclohexan/Essigsäureethylester verwendet, wobei mit einem Essigsäureethylester Gradienten von 1-20 % über 80 Minuten gearbeitet wurde. Die Pumpgeschwindigkeit lag bei 50 mL/min. Ausbeute: 19%, 0.16 g, 0.4 mmol.

mₚ= 146.9 °C (Rekristallisation aus Dichlormethan/ Cyclohexan); ¹H NMR (400 MHz, CDCl₃) δ=6.80 (d, ⁴*J*= 1.6 Hz, 2H), 6.79 (d, ⁴*J*= 1.6 Hz, 2H), 6.26 (s, 2H, OH), 3.85 (s, 6H), 2.22 (s, 6 H); ¹³C NMR (100 MHz, CDCl₃) δ=156.52, 143.35, 130.30, 126.89, 114.96, 112.87, 56.22, 21.12; HRMS für C₁₆H₁₈O₄⁸⁰Se (ESI+) [M+Na⁺]: berechnet: 377.0268, gefunden: 377.0264; Elementaranalyse für C₁₆H₁₈O₄Se: berechnet: C: 54.40%, H: 5.14%, gefunden: C: 54.32%, H: 5.14%.

### Bis(3-chlor-6-hydroxy-5-isopropyl-2-methylphenyl)selen (21)

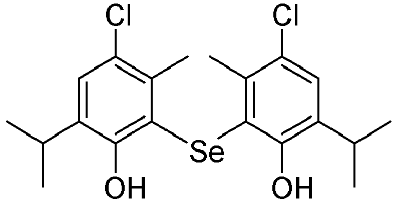

In einem 10 mL Rundkolben wurden 1.0 g Chlorthymol (54 mmol) in 7.5 mL Pyridin gelöst, mit 0.601 g Selendioxid (54 mmol) versetzt und im Ölbad auf 55 °C erhitzt. Nach sieben Tagen wurde die Reaktionslösung mit 50 mL Ethylacetat verdünnt und filtriert. Die organische Phase wurde zunächst zwei Mal mit je 40 mL 10 %iger Salzsäure, zwei Mal mit je 40 mL Wasser gewaschen und über Magnesiumsulfat getrocknet. Das, nach Destillation des Lösungsmittels erhaltene, Rohprodukt wurde mittels Säulenchromatographie gereinigt: Die Länge der Säule betrug 10 cm mit einem Durchmesser von 4 cm. Als Eluent wurde Cyclohexan/Essigsäureethylester im Verhältnis 95/5 verwendet. Das so erhaltene Produkt wurde in 20 mL Cyclohexan unter Rückfluss erhitzt. Eine gute Kristallisation konnte nur durch sehr langsames Abkühlen im Ölbad erreicht werden. Das Produkt wurde abfiltriert und mit kaltem Cyclohexan gewaschen. Das Filtrat wurde eingeengt und bei 4 °C innerhalb von zwei Tagen erneut zur Kristallisation gebracht. Nach abfiltrieren des Feststoffes wurde das Filtrat erneut eingeengt und bei 4 °C für sieben Tage zur Kristallisation gebracht. Ausbeute: 40%, 0.48 g, 1.1 mmol.

mₚ= 175.3 °C (Rekristallisation aus Cyclohexan); ¹H NMR (400 MHz, CDCl₃) δ=7.18 (s, 2H), 6.37 (s, 2H, OH), 3.21 (hept, ³*J*=6.9 Hz, 2H), 2.42 (s, 6H), 1.20 (d, ³*J*=6.9 Hz, 12H).
¹³C NMR (100 MHz, CDCl₃) δ= 152.70, 136.80, 134.16, 128.45, 126.39, 117.49, 27.96, 22.41, 20.76; HRMS für C₂₀H₂₄³⁵Cl₂O₂⁸⁰Se (ESI+) [M+Na⁺]: berechnet: 469.0216, gefudnen: 469.0269.

### Bis(3-chlor-6-hydroxy-5-methylphenyl)selen (22)

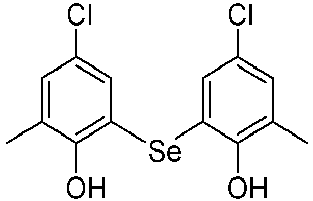

In einem 10 mL Rundkolben wurden 650 mg 4-Chlor-2-methylphenol (45 mmol) in 6.3 mL Pyridin gelöst, mit 506 mg Selendioxid (45 mmol) versetzt und im Ölbad auf 55 °C erhitzt. Nach zehn Tagen wurde die Reaktionslösung mit 50 mL Ethylacetat verdünnt und filtriert. Die organische Phase wurde zunächst zwei Mal mit je 40 mL 10%iger Salzsäure und zwei Mal mit je 40 mL Wasser gewaschen. Nach dem Trocknen über Magnesiumsulfat wurde das Lösungsmittel destillativ entfernt und das Rohprodukt mittels Säulenchromatographie gereinigt. Dabei wurde ein Säulenautomat der Firma *BÜCHI-Labortechnik GmbH,* Essen verwendet. Die Säulenlänge betrug 16 cm und der Durchmesser 6 cm. Als Eluent wurde Cyclohexan/Essigsäureethylester verwendet, wobei mit einem Essigsäureethylester Gradienten gearbeitet wurde: 1-5 % (über 15 min), 3-20 % (über 20 min), 20-60 % (20 min). Die Pumpgeschwindigkeit lag bei 50 mL/min. Das erhaltene Produkt wurde in Dichlormethan mit 5 %iger Beimischung von Methanol gelöst und mit Cyclohexan überschichtet, um eine Kristallisation an der Grenzfläche zu ermöglichen. Es wurden 0.39 g (1.0 mmol, 48%) farblose, nadelförmige Kristalle erhalten.

mₚ= 169.8 °C (Rekristallisation aus Methanol/ Cyclohexan); ¹H NMR (400 MHz, CDCl₃) δ=7.19-7.22 (m, 2H), 7.09-7.11 (m, 2H), 5.95 (s, 2H, OH), 2.25 (s, 6 H); ¹³C NMR (100 MHz, CDCl₃) δ**=**152.60, 132.24, 131.99, 126.91, 126.32, 125.57, 16.67; MS für C₁₂H₆Cl₂F₂O₂⁸⁰Se: (EI, GCMS): m/z(%): [M]^{+.}362; Elementaranalyse für C₂₀H₂₄Cl₂O₂Se: berechnet: C: 46.44%, H: 3.34%, gefunden: C: 46.42%, H: 3.48%.

### Bis(3-brom-2-hydroxy-5-methylphenyl)selen (23)

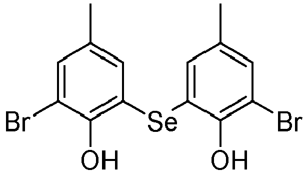

Wie in AAV1 beschrieben wurden 0.50 g 2-Brom-4-methylphenol (2.6 mmol, 1.0 eq.) zu einer Lösung von 0.16 g Selendioxid (1.4 mmol, 0.6 eq.) in 3.7 mL Pyridin gegeben und bei 65 °C für 7 Tage gerührt. Die Reaktionsmischung wurde mit 20 mL Dichlormethan verdünnt, filtriert und dreimal mit je 50 mL Salzsäure (10%) und einmal mit 50 mL Kochsalzlösung gewaschen. Nach dem Trocknen über Magnesiumsulfat wurde das Lösungsmittel unter vermindertem Druck entfernt. Der Rückstand wurde in 2 mL Dichlormethan gelöst und mit Cyclohexan überschichtet. Das Produkt wurde als leicht gelbliche Nadeln erhalten. Ausbeute: 37%, 0.23 g, 5.9 mmol.

¹H NMR (400 MHz, CDCl₃) δ=7.28-7.29 (m, 2H), 7.11-7.12 (m, 2H), 6.35 (brs, 2H, OH), 2.22 (s, 6 H); ¹³C NMR (100 MHz, CDCl₃) δ= 149.83, 134.96, 134.04, 132.20, 115.89, 109.02, 20.13; HRMS für C₁₄H₁₂⁷⁹Br₂O₂⁸⁰Se (ESI+) [M+Na⁺]: berechnet: 472.8267, gefunden: 472.8219; Elementaranalyse für C₁₄H₁₂Br₂O₂Se: berechnet: C: 37.28%, H: 2.68%, gefunden: C: 37.35%, H: 2.33%.

### Bis(3-chlor-5-fluor-2-hydroxyphenyl)selen (24)

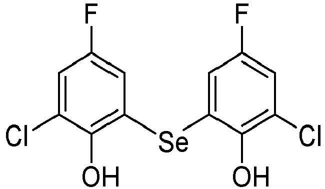

Wie in AAV1 beschrieben wurden 2.50 g 2-Chlor-4-fluorophenol (17.1 mmol, 1.0 eq.) zu einer Lösung von 1.36 g Selendioxid (10.2 mmol, 0.6 eq.) in 7.7 mL Pyridin gegeben und bei 85 °C für 48 Stunden gerührt. Anschließend wurde die Reaktionslösung mit 150 mL Ethylacetat verdünnt, filtriert und drei Mal mit je 100 mL Salzsäure (10%) und einmal mit 100 mL Kochsalzlösung gewaschen. Nach dem Trocknen über Magnesiumsulfat wurde das Lösungsmittel unter vermindertem Druck destilliert. Nicht umgesetztes Edukt wurde unter vermindertem Druck (10⁻¹ mbar) entfernt und der Rückstand säulenchromatographisch gereinigt (Laufmittel: Cyclohexan/ Ethylacetat mit einem Gradienten von 95:5 auf 80:20). Ausbeute: 64%, 2.027 g, 5.4 mmol.

mₚ= 147.6 °C (Rekristallisation aus Cyclohexan); ¹H NMR (400 MHz, CDCl₃) δ=7.13 (dd, ³*J*=7.7 Hz, ⁴*J*=2.9 Hz, 2H), 6.97 (dd, ³*J*=7.5. Hz, ⁴*J*=2.9 Hz, 2H), 5.35 (brs, 2H, OH); ¹³C NMR(100 MHZ, CDCl₃)) δ=155.94 (d, ¹*J*= 245.9 Hz), 147.88 (d, ⁴*J*= 3.0 Hz), 120.35 (d, ³*J*= 10.5 Hz), 119.99 (d, ²*J*= 23.9 Hz), 117.88 (d, ²*J*= 26.2 Hz), 116.53 (d, ³*J*= 8.0 Hz).
MS für C₁₂H₆Cl₂F₂O₂⁸⁰Se: (EI, GCMS): m/z(%): [M]⁺˙ 370; Elementaranalyse für C₁₂H₆Cl₂F₂O₂Se: berechnet: C: 38.95%, H: 1.63%, found: C: 38.93%, H: 1.60%.

### Bis(3,5-difluoro-2-hydroxyphenyl)selen (25)

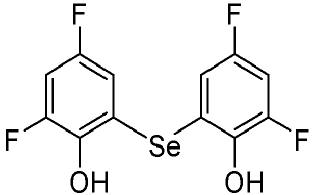

Wie in AAV1 beschrieben wurden 6.00 g 2,4-Difluorophenol (49.5 mmol, 1.0 eq.) zu einer Lösung von 3.30 g Selendioxid (29.7 mmol, 0.6 eq.) in 70 mL Pyridin gegeben und bei 85 °C für 8 Tage gerührt. Anschließend wurde die Reaktionslösung mit 150 mL Ethylacetat verdünnt, filtriert und drei Mal mit je 100 mL Salzsäure (10%) und einmal mit 100 mL Kochsalzlösung gewaschen. Nach dem Trocknen über Magnesiumsulfat wurde das Lösungsmittel unter vermindertem Druck destilliert und der erhaltene Rückstand säulenchromatographisch gereinigt (Laufmittel: Cyclohexan/ Ethylacetat mit einem Gradienten von 99:1 bis 0:100). Ausbeute: 40%, 3.34 g, 0.9 mmol.

mₚ= 130.7 °C; ¹H NMR (400 MHz, CDCl₃) δ=6.93-6.84 (m, 4H), 5.97 (bs, 2H, OH); ¹³C NMR(100 MHZ, CDCl₃) δ=155.59 (dd, ¹*J*=245.4 Hz, ³*J*=10.8 Hz), 150.04 (dd, ¹*J*=248.5 Hz ³*J*=11.6 Hz), 140.39 (dd, ²*J*=13.6 Hz, ⁴*J*=2.4 Hz), 116.95 (d, ³*J*=8.4Hz), 116.25 (dd, ²*J*=23.6 Hz, ⁴*J*=3.7 Hz), 105.72 (dd, ²*J*=27.0 Hz, ²*J*=22.2 Hz); MS für C₁₂H₆F₄O₂⁸⁰Se: (EI, GCMS): m/z(%): [M]⁺˙338; Elementaranalyse für C₁₂H₆F₄O₂Se: berechnet: C: 42.75%, H: 1.79%, gefunden: C: 42.63%, H: 1.76%.

### Bis(2,4,5-trimethoxyphenyl)selen (26)

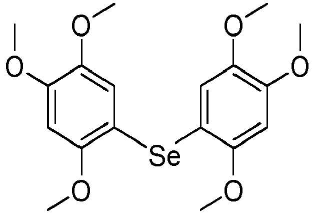

Wie in AAV2 beschrieben 2.95 g Selendioxid (26.6 mmol, 0.6 eq.) zu einer Lösung aus 10.00 g 1,2,4-Trimethoxybenzen (48.4 mmol, 1.0 eq.) in 67 mL Essigsäure gegeben. Die Lösung wurde 7 Stunden bei 85 °C gerührt. Anschließend wurden 500 mL Ethylacetat hinzugefügt, filtriert und die organische Phase drei Mal mit je 100 mL Kochsalzlösunggewaschen. Nach Trocknen über Magnesiumsulfat wurde das Lösungsmittel unter vermindertem Druck destilliert und der Rückstand säulenchromatographisch aufgereinigt (Laufmittel: Cyclohexan/ Ethylacetat 95:5). Ausbeute: 42%, 4.06 g, 48.0 mmol.

mₚ= 93.8 °C (recrystallized from cyclohexane); ¹H NMR (400 MHz, CDCl₃) δ=6.75 (s, 2H), 6.56 (s, 2H), 3.89 (s, 6H), 3.84 (s, 6H), 3.69 (s, 6 H); ¹³C NMR (100 MHz, CDCl₃) δ=151.25, 148.81, 142.90, 118.93, 115.33, 98.37, 56.91, 56.56, 56.13; HRMS for C₁₈H₂₂O₆⁸⁰Se (ESI+)
[M+Na⁺]: calc.: 437.0479, found: 437.0410; Elemental anal. for C₁₈H₂₂O₆Se: calc.: C: 52.31%, H: 5.86%, found: C: 51.97%, H: 6.08%.

### Bis(3,4-dimethoxy-6-methylphenyl)selen (27)

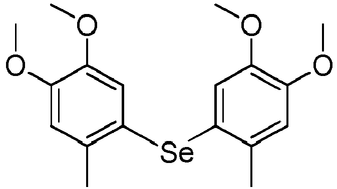

In einem 25 mL Rundkolben wurden 1.00 g 3,4-Dimethoxytoluol (6.5 mmol) in 9 mL Essigsäure gelöst, mit 0.40 g Selendioxid (3.6 mmol) versetzt und im 85 °C heißen Ölbad erwärmt. Nach 12 Tagen wurde das Reaktionsgemisch filtriert, das Filtrat mit Dichlormethan verdünnt und mit gesättigter Natriumchlorid-Lösung gewaschen. Die organische Phase wurde über Magnesiumsulfat getrocknet und das Lösungsmittel unter vermindertem Druck destilliert. Das Rohprodukt wurde mittels Säulenchromatographie auf gereinigt. Dabei wurde ein Säulenautomat der Firma *BÜCHI-Labortechnik GmbH,* Essen verwendet. Die Säulenlänge betrug 16 cm und der Durchmesser 6 cm. Als Eluent wurde Cyclohexan/Essigsäureethylester verwendet, wobei mit einem Essigsäureethylester Gradienten gearbeitet wurde: 0 % (über 5 min), 1-5 % (über 5 min), 5-10 % (über 8 min), 10-20 % (8 min), 20-40 % (10 min), 40-100 % (10 min). Die Pumpgeschwindigkeit lag bei 100 mL/min. Ausbeute: 51%, 0.637 g, 1.7 mmol.

mₚ= 85.7 °C (Rekristallisation aus Cyclohexan); ¹H NMR (400 MHz, CDCl₃) δ= 6.77 (s, 2H), 6.76 (s, 2H), 3.86 (s, 6H), 3.70 (s, 6H), 2.34 (s, 6H); ¹³C NMR (100 MHz, CDCl₃) δ=148.70, 147.54, 132.50, 121.55, 116.51, 113.47, 56.16, 56.07, 21.96; HRMS für C₁₈H₂₂O₄⁸⁰Se (ESI+) [M+Na⁺]: berechnet: 405.0581, gefunden: 405.0584; Elementaranalyse für C₁₈H₂₂O₄Se: berechnet: C: 56.70%, H: 5.82%, gefunden: C: 56.60%, H: 5.85%.

### Bis(6-methyl-2,3,4-trimethoxyphenyl)selen (28)

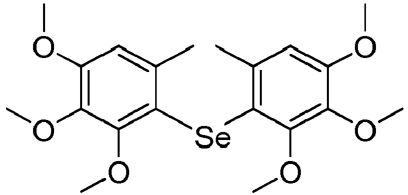

In einem 25 mL Rundkolben wurden 0.80 g 3,4,5-Trimethoxytoluol (4.3 mmol) in 6 mL Essigsäure gelöst, mit 0.27 g Selendioxid (2.4 mmol) versetzt und im 85 °C heißen Ölbad erwärmt. Nach 12 Tagen wurde das Reaktionsgemisch filtriert, das Filtrat mit Dichlormethan verdünnt und mit gesättigter Natriumchlorid-Lösung gewaschen. Die organische Phase wurde über Magnesiumsulfat getrocknet und das Lösungsmittel unter vermindertem Druck destilliert. Das Rohprodukt wurde mittels Säulenchromatographie gereinigt. Die Säulenlänge betrug 24 cm mit einem Durchmesser von 3 cm. Als Laufmittel wurde Cyclohexan/Ethylacetat im Verhältnis 9:1 verwendet. Ausbeute: 41%, 0.40 g, 0.9 mmol.

¹H NMR (400 MHz, CDCl₃) δ= 6.54 (s, 2H), 3.82 (s, 6H), 3.78 (s, 6H), 3.59 (s, 6H), 2.37 (s, 6H); ¹³C NMR (100 MHz, CDCl₃) δ=154.19, 153.06, 140.44, 137.07, 118.21, 109.17, 60.86, 60.60, 56.00, 23.52; HRMS für C₂₀H₂₆O₆⁸⁰Se (ESI+) [M+Na⁺]: berechnet: 465.0792, gefunden: 465.0780; Elementaranalyse für C₂₀H₂₆O₆Se: berechnet: C: 54.42%, H: 5.94%, gefunden: C: 54.55%, H: 5.92%.

## Patentansprüche

1. Selenodiaryl der allgemeinen Formel I zur Anwendung als Mikrobizid oder als Arzneimittel, 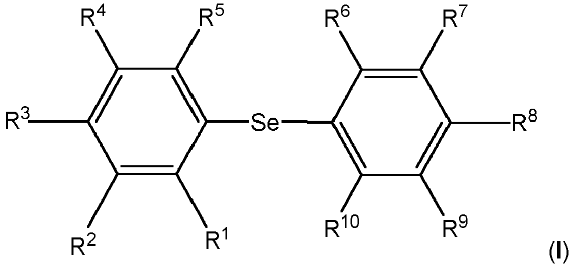 worin R¹, R², R³, R⁴, R⁵, R⁶, R¹, R⁸, R⁹ und R¹⁰ jeweils unabhängig voneinander ausgewählt sind aus: -H, -OH, -(C₁-C₁₂)-Alkyl, -O-(C₁-C₁₂)-Alkyl, -(C₆-C₂₀)-Aryl, -O-(C₆-C₂₀)-Aryl, -Halogen, -S-Alkyl, -S-Aryl, -CO-(C₁-C₁₂)-Alkyl, -CO-(C₆-C₂₀)-Aryl,
-( C₁-C₁₂)-Alkyl-O-(C₁-C₁₂)-Alkyl, -(C₁-C₁₂)-Alkyl-(C₆-C₂₀)-Aryl,
-(C₁-C₁₂)-Alkyl-O-(C₆-C₂₀)-Aryl, -(C₆-C₂₀)-Aryl-O-(C₆-C₂₀)-Aryl,
-(C₆-C₂₀)-Aryl-(C₁-C₁₂)-Alkyl, -(C₁-C₁₂)-Alkyl-O-(C₁-C₁₂)-Alkyl-(C₆-C₂₀)-Aryl,
-(C₁-C₁₂)-Alkyl-(O-(C₁-C₁₂)-Alkyl)ₓ-(C₁-C₁₂)-Alkyl, mit x = 1 bis 3, -COOH, -SO₃H, -CN, wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, -Halogen, -Cyano, -Formyl, -Acyl oder -Alkoxycarbonyl.

2. Selenodiaryl nach Anspruch 1 zur Anwendung als Antimykotikum.

3. Selenodiaryl nach Anspruch 2 zur Anwendung als Antimykotikum gegenüber mindestens einem Pilz der ausgewählt ist aus der Gattung *Mucor, Phytophthera, Paecilomyces, Penicillium, Botrytis, Magnaporthe* und/oder *Candida.*

4. Selenodiaryl nach einem der Ansprüche 1 bis 3, wobei das Selenodiaryl der allgemeinen Formel **I** eine fungistatische und/oder fungizide Wirkung aufweist.

5. Selenodiaryl nach einem der Ansprüche 1 bis 4, wobei das Selenodiaryl der allgemeinen Formel **I** in einer Zusammensetzung mit einem Gehalt größer gleich 0,001 Gew.-% bis 100 Gew.-% vorliegt, wobei die Gesamtzusammensetzung 100 Gew.-% beträgt.

6. Selenodiaryl nach einem der Ansprüche 1 bis 5, wobei das Selenodiaryl der allgemeinen Formel I ausgewählt ist aus der allgemeinen Formel **II** 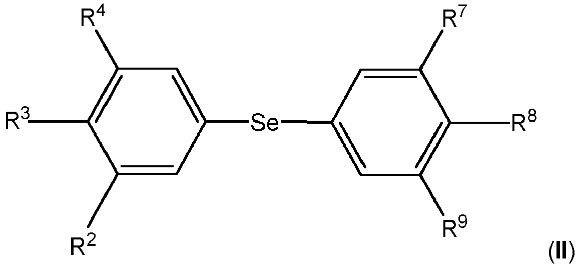 worin R², R⁴, R⁷ und R⁹ jeweils unabhängig voneinander ausgewählt sind aus: -H, -OH, -(C₁-C₁₂)-Alkyl, -O-(C₁-C₁₂)-Alkyl, -(C₆-C₂₀)-Aryl, -O-(C₆-C₂₀)-Aryl, -Halogen,
-(C₁-C₁₂)-Alkyl-O-(C₁-C₁₂)-Alkyl, -(C₁-C₁₂)-Alkyl-(C₆-C₂₀)-Aryl,
-(C₁-C₁₂)-Alkyl-O-(C₆-C₂₀)-Aryl, -(C₆-C₂₀)-Aryl-O-(C₆-C₂₀)-Aryl,
-(C₆-C₂₀)-Aryl-(C₁-C₁₂)-Alkyl, -(C₁-C₁₂)-Alkyl-O-(C₁-C₁₂)-Alkyl-(C₆-C₂₀)-Aryl,
-(C₁-C₁₂)-Alkyl-(O-(C₁-C₁₂)-Alkyl)x-O-(C₁-C₁₂)-Alkyl, mit x = 1 bis 3, wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Halogen, und,
worin R³ und R⁸ jeweils unabhängig voneinander ausgewählt sind aus: -OH, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkyl- und Arylgruppen jeweils unsubstituiert sind.

7. Selenodiaryl nach einem der Ansprüche 1 bis 5, wobei das Selenodiaryl der Formel **I** ausgewählt ist aus der allgemeinen Formel **Ia** 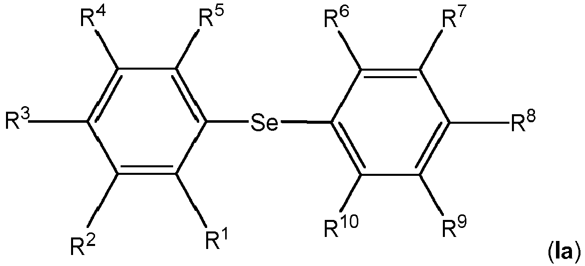 worin R¹, R³, R⁵, R⁶, R⁸ und R¹⁰ jeweils unabhängig voneinander ausgewählt sind aus: -H, -OH, -(C₁-C₁₂)-Alkyl, -O-(C₁-C₁₂)-Alkyl, -Halogen,
-(C₁-C₁₂)-Alkyl-O-(C₁-C₁₂)-Alkyl, -(C₁-C₁₂)-Alkyl-O-(C₁-C₁₂)-Alkyl-(C₆-C₂₀)-Aryl, -(C₁-C₁₂)-Alkyl-(O-(C₁-C₁₂)-Alkyl)x-O-(C₁-C₁₂)-Alkyl, mit x = 1 bis 3, wobei die Alkylgruppen jeweils unabhängig unsubstituiert oder substituiert sind, wobei substituierte -(C₁-C₁₂)-Alkylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Halogen, und,
worin R², R⁴, R⁷ und R⁹ jeweils unabhängig voneinander ausgewählt sind aus: -H, -OH, -(C₁-C₄)-Alkyl, -O-(C₁-C₄)-Alkyl, -Halogen, wobei die Alkylgruppen jeweils unsubstituiert oder substituiert sind, wobei substituierte -(C₁-C₄)-Alkylgruppen mindestens einen Substituenten aufweisen und der mindestens eine Substituent jeweils unabhängig ausgewählt ist aus Halogen.

8. Selenodiaryl nach einem der Ansprüche 1 bis 5, wobei das Selenodiaryl der Formel I ausgewählt ist aus der allgemeinen Formel **Ib** 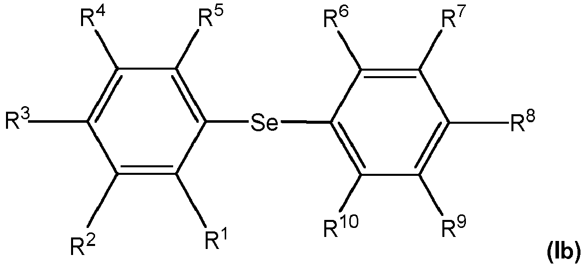 worin R¹, R², R³, R⁴, R⁵, R⁶, R⁷, R⁸, R⁹ und R¹⁰ jeweils unabhängig voneinander ausgewählt sind aus: -H, -OH, -Halogen, -(C₁-C₄)-Alkyl, -O-(C₁-C₄)-Alkyl, wobei die Alkylgruppen jeweils unabhängig unsubstituiert oder substituiert sind, wobei substituierte -(C₁-C₄)-Alkylgruppen mindestens einen Substituenten aufweisen der ausgewählt ist aus -Halogen.

9. Selenodiaryl nach einem der Ansprüche 1 bis 8, wobei das Selenodiaryl ausgewählt ist aus
a) Bis(3,5-dimethyl-4-hydroxyphenyl)selen, Bis(3-chlor-5-fluor-2-hydroxyphenyl)selen, 3,5-difluoro-2-hydroxyphenyl)selen, oder
b1) Bis(3,5-dimethyl-2-hydroxyphenyl)selen, Bis(5-*tert*-butyl-2-hydroxy-3-methylphenyl)selen, Bis(2-hydroxy-3-methoxy-5-methylphenyl)selen, Bis(3-chlor-6-hydroxy-5-isopropyl-2-methylphenyl)selen, Bis(3-chlor-6-hydroxy-5-methylphenyl)selen, Bis(3-brom-2-hydroxy-5-methylphenyl)selen, Bis(2,4,5-trimethoxyphenyl)selen, oder
b2) Bis(2-hydroxy-3,5,6-trimethylphenyl)selen, Bis(3,4-dimethoxy-6-methylphenyl)selen, Bis(6-methyl-2,3,4-trimethoxyphenyl)selen.

10. Selenodiaryl nach einem der Ansprüche 1 bis 9 zur Verwendung als
a) mikrobizides Arzneimittel zur Prävention und/oder Behandlung von Pilzinfektionen und/oder Pilzkrankheiten bei Mensch oder Tier, oder
b) Mikrobizid zur Prävention und/oder Behandlung von Pilzinfektionen und/oder Pilzkrankheiten bei Pflanzen eingesetzt wird.

11. Selenodiaryl einem der Ansprüche 1 bis 10 zur Anwendung bei einer Prävention und/oder Bekämpfung eines mikrobiziden Befalls organischer Materialien, anorganischer Materialien oder Hybridmaterialien umfassend organische und anorganische Materialien, insbesondere zur Prävention und/oder Bekämpfung eines mikrobiziden Befalls mineralischer Werkstoffe, organischer Werkstoffe, Baumaterialien, Textilien, Lacken, Metallen, Legierungen, Holz, natürlichen Polymeren, synthetischen Polymeren, Lebensmitteln, Futtermitteln und/oder elektronischer Bauteile.

12. Zusammensetzung umfassend mindestens ein Selenodiaryl der Formel **I** zur Anwendung als Mikrobizid oder Arzneimittel, insbesondere als mikrobizides Arzneimittel, 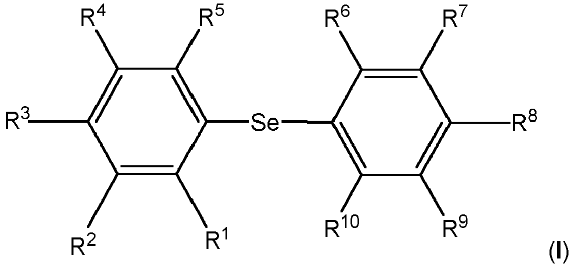 worin R¹, R², R³, R⁴, R⁵, R⁶, R⁷, R⁸, R⁹ und R¹⁰ jeweils unabhängig voneinander ausgewählt sind aus: -H, -OH, -(C₁-C₁₂)-Alkyl, -O-(C₁-C₁₂)-Alkyl, -(C₆-C₂₀)-Aryl, -O-(C₆-C₂₀)-Aryl, -Halogen, -S-Alkyl, -S-Aryl, -CO-(C₁-C₁₂)-Alkyl, -CO-(C₆-C₂₀)-Aryl, -(C₁-C₁₂)-Alkyl-O-(C₁-C₁₂)-Alkyl, -(C₁-C₁₂)-Alkyl-(C₆-C₂₀)-Aryl,
-(C₁-C₁₂)-Alkyl-O-(C₆-C₂₀)-Aryl, -(C₆-C₂₀)-Aryl-O-(C₆-C₂₀)-Aryl,
-(C₆-C₂₀)-Aryl-(C₁-C₁₂)-Alkyl, -(C₁-C₁₂)-Alkyl-O-(C₁-C₁₂)-Alkyl-(C₆-C₂₀)-Aryl,
-(C₁-C₁₂)-Alkyl-(O-(C₁-C₁₂)-Alkyl)x-O-(C₁-C₁₂)-Alkyl, mit x = 1 bis 3, -COOH, -SO₃H, -CN, wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, -Halogen, -Cyano, -Formyl, -Acyl oder -Alkoxycarbonyl.

13. Zusammensetzung nach Anspruch 12, **dadurch gekennzeichnet, dass** die Zusammensetzung vorliegt
i) als Lösung, Injektion, Infusion, Tinktur, Tropflösung, Suspension, Emulsion, Dispersion, oder
ii) als Pulver, Lyophilisat, Tablette, Granulat, Filmtablette, Dragee, Kapsel, Brausetablette, Puder oder Seife, oder
iii) als Spray, Aerosol, Emulsion, Salbe, Paste und Kapsel, Gel, Schaum oder Creme.

14. Zusammensetzung nach Anspruch 12 oder 13, **dadurch gekennzeichnet, dass** die Zusammensetzung eine Formulierung zur Anwendung als Arzneimittel, Kosmetikum, Medizinprodukt, Pflanzenschutzmittel, Desinfektionsmittel, Biozid, Werkstoffschutzmittel; Nahrungsergänzungsmittel ist oder Zusammensetzung umfassend das Selenodiaryl der allgemeinen Formel **I** als Additiv zur Anwendung als Mikrobizid.

15. Verwendung eines Selenodiaryls der allgemeinen Formel **I** zur Herstellung einer mikrobizid wirkenden nicht medizinischen Ausrüstung, zur Herstellung einer mikrobizid wirkenden nicht medizinischen Zusammensetzung und/oder zur mikrobizid wirkenden Funktionalisierung oder Ausrüstung von funktionalisierbaren Substraten.
